# EUROPEAN PATENT SPECIFICATION

(11) **EP 4 044 985 B1**
(45) Date of publication and mention of the grant of the patent: **30.09.2026**
(21) Application number: 20789645.7
(22) Date of filing: 19.10.2020
(51) Int. Cl.: A61H 11/00, A61H 23/02, A61N 2/00, A61N 2/02, A63B 23/18, A63B 23/00

(54) **RESPIRATION PROMOTING APPARATUS AND USE THEREOF**
ATMUNGSFÖRDERUNGSGERÄT UND DESSEN VERWENDUNG
APPAREIL FACILITANT LA RESPIRATION ET UTILISATION CORRESPONDANTE

(30) Priority: 18.10.2019 CH 13262019
(43) Date of publication of application: 24.08.2022
(73) Proprietor: Stimit AG, 2503 Biel (CH)
(72) Inventor: SCHNELL, Pascal André, 4252 Bärschwil (CH); DEGEN, Thomas, 8903 Birmensdorf (CH); FENGELS, Dirk, 6037 Root (CH); MÜLLER-BRUHN, Ronja, 5210 Windisch (CH)
(74) Representative: Latscha Schöllhorn Partner AG
(86) International application number: PCT/EP2020/079402
(87) International publication number: WO 2021/074453

(56) References cited:
- WO-A1-2017/055471
- WO-A1-2019/154839
- DE-U1- 29 812 986
- GB-A- 2 587 392
- US-A1- 2016 310 730
- T. SIMILOWSKI ET AL: "Cervical magnetic stimulation: a new painless method for bilateral phrenic nerve stimulation in conscious humans", JOURNAL OF APPLIED PHYSIOLOGY, vol. 67, no. 4, 1 October 1989 (1989-10-01), US, pages 1311 - 1318, XP055655326, ISSN: 8750-7587, DOI: 10.1152/jappl.1989.67.4.1311
- THOMAS SIMILOWSKI: "Respiratory applications of magnetic stimulation", PROCEEDINGS OF THE ANNUAL INTERNATIONAL CONFERENCE OF THE IEEE ENGINEERING IN MEDICINE AND BIOLOGY SOCIETY : PARIS, FRANCE, OCTOBER 29 - NOVEMBER 1, 1992, IEEE, NEW YORK, NY, USA, 29 October 1992 (1992-10-29), pages 1414 - 1416, XP031862394, ISBN: 978-0-7803-0785-8, DOI: 10.1109/IEMBS.1992.5761855
- HOVEY C ET AL: "THE GUIDE TO MAGNETIC STIMULATION", 21 July 2006 (2006-07-21), pages 1 - 44, XP008148930, Retrieved from the Internet <URL:http://www.icts.uci.edu/neuroimaging/GuidetoMagneticStimulation2008.pdf>

## Description

### Technical Field

The present invention relates to an electro-magnetic respiration promoting apparatus for stimulating Phrenic nerves of a human or animal being and a use of the respiration promoting apparatus in a method for promoting respiration of a human or animal. More particularly, the present invention relates to a non-invasive electro-magnetic respiration promoting apparatus using a first coil winding unit for stimulating a first Phrenic nerve and a second coil winding unit for stimulating a second Phrenic nerve.

### Background Art

In medicine, it is known that for many purposes it is beneficial to activate a target tissue of a patient using stimulation by electro-magnetic fields. For achieving such activation of tissues in a patient's body, it is known to directly stimulate the tissue or to indirectly activate the tissue via stimulation of specific parts of the neural system. For example, the target tissue being a muscular tissue can be activated by providing electric pulses directly to the muscular tissue or to nerves associated to the muscular tissue.

In critical care units of hospitals it may be desired to activate the diaphragm of ventilated patients in order to prevent drawbacks of disuse of the diaphragm. It was shown that disuse atrophy of diaphragm muscle fibres occurs already in the first 18-69 hours of mechanical ventilation, and the muscle fibre cross-sections decreased by more than 50% in this time. Thus, it is aimed to activate the diaphragm repeatedly while the patient is given artificial or mechanical respiration such that the functioning of the diaphragm can be upheld, or to activate the diaphragm at least during the weaning period to support effective restoration of independent respiratory function.

It is known that the diaphragm can be activated by stimulating the two Phrenic nerves, e.g., at the neck of a patient. In this context, US 2016/0310730 A1 describes an apparatus for reducing ventilation induced diaphragm disuse in a patient receiving ventilation support from a mechanical ventilator. The apparatus includes an electrode array of first and second types and comprises a plurality of electrodes configured to stimulate a Phrenic nerve of the patient. At least one controller identifies a type of electrode array and generates a stimulus signal for stimulating a Phrenic nerve of the patient based upon the identity of the electrode type. Such electrode-based stimulation is not very robust to patient movements or relocations, and the possible stimulation depth can be significantly limited by bones or fatty tissue. Also, electrode stimulation is reported to be more painful for the patient than electro-magnetic stimulation.

In one example, the electrode arrays are configured as surface electrode arrays embedded in adhesive electrode patches to be placed on each side of a patient's neck near the areas where the Phrenic nerves are located. Thus, the electrode arrays are electrically and mechanically coupled to the skin of the patient independent from each other. Therefore, the electrode arrays cover portions of a patient's surface and may interfere with other applications required for the treatment of the patient like for example the intubation into the trachea. In case the electrode arrays need to be taken off and replaced at a later point in time, the system needs to be fully re-calibrated according the new locations of the electrode arrays to avoid undesired co-stimulation of tissue surrounding the Phrenic nerves.

In practice, also electro-magnetic stimulators are used to activate a target tissue, which are based on the principle of electro-magnetic induction. A strong current pulse (typically a monophasic or biphasic current pulse) flows through a coil winding, which produces a strong, transient magnetic field. The current pulses cause a changing magnetic field that for example alters according to the phases of the current pulses. The changing magnetic field induces a corresponding electric field, which in turn depolarizes neuronal membranes, leading to action potentials through one or more nerves. Coil windings are usually designed towards generating electric field distribution curves of the induced field, often having an electric field peak (area with maximum electric field strength) or an electric field area, which is stronger than other fields in other areas. Thus, an electric field distribution curve can generate activation pulses, which are effective periodically at time intervals, and electric field peaks or strong field areas may alternate with low field areas in the distribution curve.

However, when two target nerves inside the body in a close distance to each other, like the two Phrenic nerves, shall be stimulated simultaneously, the coil winding systems in today's stimulators used for electro-magnetic stimulation have significant limitations. De-central coils have been designed for this purpose producing de-centralized fields and have been described for instance in DE 10 2007 013531 A1. However, typically two separate stimulator devices are necessary, the electromagnetic fields of the two devices can interfere with each other, and body constraints may not allow positioning coils windings of the two devices in parallel to make use of the de-centralized design. Especially in the neck region, positioning coil windings longitudinal to the neck would force the user to choose significantly smaller coil winding sizes because the chin and chest constrain space for the coils to be placed. Patent document WO2019154839A1 discloses electro-magnetic stimulation of a phrenic nerve in a patient to improve respiratory function.

Therefore, there is a need for a non-invasive respiration promoting apparatus and use thereof and a non-invasive method for promoting respiration of a patient, which allow for efficient stimulation of both Phrenic nerves, overcome space constraints, avoid co-stimulation effects of tissue in the vicinity of the Phrenic nerves, are simple to apply at a patient as well as convenient and pain-free for the patient.

### Disclosure of the Invention

According to the invention this need is settled by a respiration promoting apparatus as it is defined by the features of independent claim 1. Preferred embodiments are subject of the dependent claims.

In particular, the invention provides a respiration promoting apparatus to coordinately stimulate two Phrenic nerves of a patient for activating a diaphragm of the patient. The respiration promoting apparatus comprises a first coil unit having a first forward face configured to be positioned at the patient to stimulate a first Phrenic nerve of the two Phrenic nerves of the patient, and a second coil unit having a second forward face configured to be positioned at the patient to stimulate a second Phrenic of the patient. It further comprises a bracket structure coupled to the first coil unit and the second coil unit. The bracket structure has a third forward face configured to be positioned at the patient and is adjustable such that positions and orientations of the first coil unit and of the second coil unit are adapted. Thus, by changing a configuration for adjusting the bracket structure the position of the first coil unit and the second coil unit relative to the patient and relative to each other can be adapted to specific treatment requirements of a specific patient for example by choosing differing distances between the coil units and a patient's body. Further, the orientation of first and second coil unit relative to each other and the orientation of the coil units relative to the patient can be adapted for example by choosing differing angle adjustments for the first and the second coil units.

The first and the second coil unit may provide bilateral stimulations of the phrenic nerves. This is useful for diaphragm activations, particularly for mechanically ventilated patients to avoid diaphragm atrophy and ventilator-related side effects, for patients lacking respiratory stimulus (e.g. paraplegic patients, patients in need for reanimation, sleep apnea patients second degree), for diagnostic purposes to determine diaphragm strength of patients, and for many other applications where it is useful to stimulate the diaphragm.

The respiration promoting apparatus of the invention is configured to be arranged at a defined location of the patient, when the first forward face, the second forward face and the third forward face are positioned at the patient. The term, "positioned at" as used herein can relate to being located at a patient's body by contacting it. For example, the three forward faces of the stimulation device can be positioned at the body by lying on it. When being positioned at the body, the three forward faces can form three support points and determine defined contact points on the body. Like this, the respiration promoting apparatus can be stably and precisely positioned with respect to the patient's Phrenic nerves. Further, by choosing a specific target configuration of the bracket structure and specific contact points on the patient's body a defined location of the respiration promoting apparatus with respect to the patient is determined.

The respiration promoting apparatus according to the invention can easily be adjusted to specific individual needs of a patient like to the characteristics of a patient's body or for coordination with other medical devices for treatment of the patient. At the same time the electro-magnetic field generated for stimulating the Phrenic nerves can be optimized for depolarizing the membrane of the Phrenic nerves and for avoiding co-stimulation of other tissues.

Although not part of the claimed invention, the present disclosure also refers to a method of promoting respiration of a patient comprising obtaining a respiration promoting apparatus as described herein. The method further includes adjusting the bracket structure of the respiration promoting apparatus such that the first forward face of the first coil unit can be positioned at and oriented to the patient to stimulate a first Phrenic nerve of the patient and the second forward face of the second coil unit can be positioned at and oriented to the patient to stimulate a second Phrenic nerve of the patient, the third forward face of the bracket structure is positioned at the patient's body. Then, the respiration promoting apparatus is arranged at a defined location of the patient such that the first forward face, the second forward face and the third forward face provided at the bracket structure are in contact with the patient, and current is coordinatedly provided to the first coil unit and the second coil unit such that the first Phrenic nerve and the second Phrenic nerve are stimulated and a diaphragm of the patient is homogeneously activated.

Like this, the respiration promoting apparatus may be adjusted for being applied to the patient's neck or chest to generate a localized electro-magnetic field in a localized gap next to the sternocleidomastoid muscle or at a location with minimum overlying muscular structures, where stimulation of overlying muscular structures can be avoided. Thus, the Phrenic nerve at a depth of about 2 - 4 cm inside a patient's neck can be stimulated by generating a coordinated electro-magnetic field in the first and second coil unit. Advantageously, the electro-magnetic field does not extend field amplitudes over a certain threshold level. Thus, the electro-magnetic field does not extend too far beyond the target area of the Phrenic nerves and therefore does not affect or co-stimulate the larynx and the sternocleidomastoid muscle.

Preferably, the first coil unit and the second coil unit and/or the bracket structure may comprise a vacuum device for fixing their respective forward face to the patient using a vacuum. The vacuum device could be realized for example by a suction cup that generates a slight under pressure when pressed on a body surface of the patient. The suction cup can simple be realized by a shallow hollow or recession in a surface of a coil unit housing. A forward face could be fixed on the body with a slight vacuum. The vacuum device could for example be created by a recess that reaches into a free space in the coil windings. At a diameter of the coil winding, the area on the skin would be large, and could provide enough negative pressure for a high adhesion on the body surface. Incidentally, in case of slipping, the vacuum would be interrupted, which in turn could be a detectable parameter to mitigate the risk of coil position loss or change with respect to the body.

Preferably, the backward face and/ or lateral faces of the first coil unit and the backward face of the second coil unit are shielded. By shielding the coil units it can be prevented that persons and/or equipment are exposed to an electromagnetic field backwardly diverging from the coil units. Advantageously, lateral faces of the first coil unit and the second coil unit are shielded. The lateral face can particularly be formed by a circumferential side of a coil unit connecting the forward face and the backward face.

A shielding for the backward and/or lateral faces can be provided for example by a passive shield such as a u-Metal, a passive coil winding, or the like. Preferably, an active shielding member is provided and configured to shield the backward faces and/ or lateral faces of the first coil unit and the second coil unit. The active shielding member can for example comprise coil windings configured to cancel out a field and/or change a field direction generated by the first coil unit and the second coil unit. For example, it can comprise one or more coil windings with a synchronized current in opposing direction of an underlying first or second coil winding of the first and second coil unit.

Shielding the backward faces and lateral faces of the first and second coil units assists in reducing unwanted co-stimulation of tissue in the vicinity of the Phrenic nerves and negative side effects associated therewith. Also, an overly focused electro-magnetic field for stimulating the Phrenic nerves can be avoided.

In a preferred embodiment of the respiration promoting apparatus of the invention, the bracket structure comprises a first leg portion, a second leg portion and a hinge portion connecting the first leg portion and the second leg portion to each other. The leg portions can be realized for example by an elongated stiff element, like a bar or a rod. The hinge portion can be realized as any articulated joint that connects the first and second leg portions in an articulated manner, for example a swivel joint or a ball joint. The first coil unit is coupled to the first leg portion and the second coil unit is coupled to the second leg portion. For example, the first and second coil units are coupled to one end of first and second leg elements, respectively, and the hinge portion is arranged at an opposing end of these elements and flexibly couples the first and second leg elements. A distance between the first coil unit and the second coil unit can be adjusted by pivoting the first leg portion and the second leg portion relative to each other about the hinge portion. Thus, the bracket structure configuration can easily be adjusted by selecting a pivoting position of the first leg portion relative to the second leg portion, and the position and orientation of the first and second coil units can be adapted for applying the respiration promoting apparatus at a defined location of a patient.

Preferably, the hinge portion of the bracket structure comprises the third forward face. Thus, when the respiration promoting apparatus is positioned at the patient and the third forward face lies on the patient, the hinge portion can serve as a support point for aligning the first and the second coil unit relative to the patient, particularly at the neck of the patient and relative to the patient's Phrenic nerves. Also, the hinge portion can serve as a symmetry axis or plane, which facilitates coordinated adaption of the position and orientation of the coil units by analogue alignment of the first and second leg portion. Thus, a customized positioning of the respiration promoting apparatus on the patient's body is possible.

Advantageously, the first leg portion and/or the second leg portion of the bracket structure are curved such that in any pivoting position of the first leg portion and the second leg portion relative to each other a free space is provided between the hinge portion and the first coil unit and/or the second coil unit. Preferably, both leg portions are curved and may have identical curved shapes. Thus, a free space is provided between the hinge portion and the first coil unit as well as between the hinge portion and the second coil unit.

Preferably, the first leg portion and the second leg portion of the bracket structure are curved such that a free space is provided between the patient and the first leg portion and/or between the patient and the second leg portion when the respiration apparatus is arranged at the defined location of the patient.

A curved shape of the leg portions is for example realized by bending or kinking an elongated element in one or more directions such that a middle portion of the element lies outside a straight line between both end portions of the element. Thus, the respiration promoting apparatus may keep clear a front area of a patient's neck in any chosen bracket structure configuration and any chosen pivoting position of the first and second leg portion.

By means of the curved first and second leg portions, the body of the patient is accessible via the free space. For example, this allows for having the neck of the patient accessible for tracheotomy during use of the respiration promoting apparatus. At the same time, it can be achieved that the first forward face, the second forward face and the third forward face are in contact with the patient providing for a stable three support point arrangement at a defined location of the patient. Additionally, the respiration promoting apparatus can be adapted such, that the three support points on a patient's body are selected according to convenience of the patient.

Preferably, the bracket structure comprises a first pivoting coupler extending from a first leg portion and a second pivoting coupler extending from a second leg portion, wherein the first and second leg portion may for example be realized as mentioned above. The first coil unit is mounted to the first pivoting coupler and the second coil unit is mounted to the second pivoting coupler. The first and second pivoting couplers can be identical. They can be realized with an articulation bearing that couples the first and second coil units in a moveable manner to the first and second leg portion, respectively. Advantageously, the pivoting coupler includes more than one articulation axis such that the coil unit can be moved relative to a leg portion in more than one direction. The pivoting couplers allow for adjustment of the orientation of the first coil unit and the second coil unit relative to each other and relative to the patient without moving the first and second leg portions relative to each other. Thus, the pivoting couplers provide a fine-tuning option for aligning an electro-magnetic field generated by the coil units with respect to their assigned Phrenic nerves while other body tissues are avoided by the field.

Preferably, the first pivoting coupler is pivotably mounted to the first leg portion such that the first pivoting coupler can be pivoted relative to the first leg portion about an articulation axis defined by the first leg portion, and respectively the second pivoting coupler is pivotably mounted to the second leg portion such that the second pivoting coupler can be pivoted relative to the second leg portion about an articulation axis defined by the second leg portion. The articulation axis can for example be arranged along a longitudinal axis of the leg portions. The pivoting coupler and the coil unit as a whole can be moved relative to their leg portion. In this case the pivoting coupler could be regarded being part of the coil unit. For example, the pivoting coupler is located basically on the articulation axis of the leg portion and is rotated about that axis relative to the leg portion. Thus, the coil unit basically remains in position towards the bracket structure and the patient, respectively, but changes its angle adjustment and therefore the direction of generating the electro-magnetic field.

Additionally or alternatively, the first coil unit can be pivotably mounted to the first pivoting coupler such that the first coil unit may be pivoted relative to the first pivoting coupler about an articulation axis of the first pivoting coupler, and the second coil unit can be pivotably mounted to the second pivoting coupler such that the second coil unit may be pivoted relative to the second pivoting coupler about an articulation axis of the second pivoting coupler. Accordingly, the orientation of the coil units relative to their respective pivoting coupler can be adapted. For example, the coil unit is located basically on the pivoting coupler articulation axis and is rotated about that axis relative to the pivoting coupler. Therefore, the direction of areas where electro-magnetic fields are generated by the first and second coil units of the respiration promoting apparatus can be changed without adjusting the position of the coil units relative to the patient and without adjusting the first and second leg portion relative to each other simply by adjusting the orientation of the coil units. Again, the adjustable nature of the coil units of the respiration promoting apparatus facilitates optimal stimulation of the Phrenic nerves and simplifies positioning of the apparatus at a patient.

Advantageously, the articulation axis along the first leg is essentially perpendicular to the articulation axis of the first pivoting coupler and the articulation axis along the second leg is essentially perpendicular to the articulation axis of the second pivoting coupler. Consequently, the first and the second coil units are supported by the pivoting couplers such that the areas of the first and second electro-magnetic fields generated by the coil units can be adapted to any desired direction relative to the respiration promoting apparatus. Therefore, the coil units can be small and overcome space constrains, a targeted alignment of the electro-magnetic fields can avoid co-stimulation effects of tissue in the vicinity of the Phrenic nerves and the light structure of the respiration promoting apparatus allows for convenient positioning at the patient.

In one preferred embodiment, the first pivoting coupler is Y-like or U-like shaped having a stem portion and two branch portions and the second pivoting coupler is similarly Y-like or U-like shaped having a stem portion and two branch portions. The stem portion of first pivoting coupler is mounted to the first leg portion and the stem portion of second pivoting coupler is mounted to the second leg portion. The first coil unit is mounted between the two branch portions of the first pivoting coupler and the second coil unit is mounted between the two branch portions of the second pivoting coupler. Therefore, the two branch portions of a pivoting coupler embrace the coil unit. End parts of the branch portions are arranged opposite to each other and may serve as bearings for rotatably supporting the coil unit at the pivoting coupler. A pivoting coupler articulation axis as mentioned before may for example run through the branch portions, particularly through their end parts.

In an alternative preferred embodiment, the first and the second pivoting coupler are ring-shaped comprising a ring portion with a ring opening and a stem portion, wherein a stem portion of a first pivoting coupler is mounted to the first leg portion and a stem portion of a second pivoting coupler is mounted to the second leg portion. The first coil unit is mounted in the ring opening of the first pivoting coupler and the second coil unit is mounted in the ring opening of the second pivoting coupler. The ring portion of the pivoting coupler provides a frame for holding the coil unit. A pivoting coupler articulation axis as described before can be oriented radially in the ring portion and across two opposite sections of the ring portion. The pivoting coupler may be configured such that the position of the pivoting coupler articulation axis along the ring portion may be changed. Thus, the coil unit can be turned within the ring portion. A ring-shaped pivoting coupler can provide protection and stability for the coil unit, and may provide a variety of bearing positions for the articulation axis along the circumference of the ring portion.

In another preferred embodiment of the respiration promoting apparatus of the invention, the bracket structure comprises a first frame portion, a second frame portion and a forehead support structure. Thereby, the first coil unit is coupled to the first frame portion and the second coil unit is coupled to the second frame portion, the first frame portion and the second frame portion are connected via the forehead support structure, and the forehead support structure is configured to be positioned at and to contact a forehead of the patient. The forehead support structure can establish or include the third forward face.

Such a bracket structure allows for safely and conveniently mount the apparatus to the patient and to securely supporting or holding the apparatus at an
appropriate location or position. In particular, it can be achieved that the coil units are stably located at the patient. Further, such bracket structure may allow for sophisticatedly adjusting the exact position and orientation of the coil units such that the Phrenic nerves can efficiently be stimulated. Also, such bracket structure allows for keeping a front side of the patient and, in particular, a front side of his neck and torso free and accessible. Like this, parallel treatment of the patient and treatment comfort can be enhanced.

The forehead support may be embodied in a cap- or hut-like manner to be worn by the patient such that the frame portions are correctly positioned and such that the forehead of the patient support the apparatus. Preferably, the bracket structure comprises an overhead arch having a first tangential end and a second tangential end, the first frame portion is mounted to the first tangential end of the overhead arch, and the second frame portion is mounted to the second tangential end of the overhead arch. Like this, the apparatus may be designed similar to a headphone additionally having the forehead support. Such configuration allows for an efficiently applicable safe mounting and operation of the apparatus.

Thereby, the bracket structure is configured such that a distance between the overhead arch and the first coil unit and a distance between the overhead arch and the second coil unit are adjustable. In particular, the overhead arch or the first and second frame portions may be height adjustable. Like this, the first and second coil units can be properly positioned at the neck of the patient for stimulating the Phrenic nerves.

The forehead support structure of the bracket structure preferably is configured to be adjusted in accordance with a size of the forehead of the patient. Such adjustable forehead support allows for accurately supporting the device.

Preferably, the forehead support structure of the bracket structure comprises a band configured to extend along the forehead of the patient. Such band allows for a safe and easily adjustable implementation of the forehead support.

The forehead support structure of the bracket structure further preferably comprises a pad configured to contact the forehead of the patient. Such pad, which can be the third forward face, allows for providing a convenient and safe support on the forehead of the patient.

Preferably, each of the first frame portion and the second frame portion comprises a neck position guide section to which the first coil unit and the second coil unit, respectively, is movably mounted such that the first coil unit and the second coil unit can be varyingly positioned about a neck of the patient. For example, the neck position guide section may be embodied in a rail like fashion, wherein the coil units are configured to be held such that they can be slid or moved along the neck position guide. Advantageously, the neck position guide is bent such that it may more or less follow the neck when being positioned aside the neck of the patient.

Thereby, preferably the first coil unit is mounted to the neck position guide section of the first frame portion by a first fastening member configured to be in a fixed state, in which the first coil unit is immovably fastened to the neck position guide section of the first frame portion, and in a loose state, which the first coil unit is movable relative to the neck position guide section of the first frame portion, and the second coil unit is mounted to the neck position guide section of the second frame portion by a second fastening member configured to be in a fixed state, in which the second coil unit is immovably fastened to the neck position guide section of the second frame portion, and in a loose state, which the second coil unit is movable relative to the neck position guide section of the second frame portion. By means of the first and second fastening members the position and orientation of the first coil and second units relative to each other can be blocked in a target configuration. Thus, such first and second fastening members may establish a locking mechanism allowing to preserve a configuration set to the individual situation given at a specific patient.

Preferably, the bracket structure comprises two support structures configured to support the respiration promoting apparatus on a torso of the patient. By such support structures the apparatus may be particularly securely be supported on the patient's body. It may be achieved that the apparatus contacts the body at three comparably solid portions of the body such as the forehead and the two clavicles of the patient.

Thereby, one of the two support structures preferably is comprised by the first frame portion and the other of the two support structures is comprised by the second frame portion. Like this, the frame portions themselves may be supported at the patient's body such as at his clavicles or the like.

Alternatively or additionally, one of the two support structures preferably comprises a first support bar movably connected to the first frame portion and the other of the two support structures preferably comprises a second support bar movably connected to the second frame portion. The support bars can be movable by being pivoted relative to the respective frame portion. Like this, a torso support can be provided which can be adjusted to the given situation of the specific patient. Also, such configuration allows to efficiently support the apparatus at various portions of the torso such as at the clavicles, ribs or sternum.

In a further embodiment of the respiration promoting apparatus, the first coil unit and the second coil unit are detachably coupled to the bracket structure. For example, the coil units may be detachably coupled to the pivoting coupler. Alternatively, or additionally, the pivoting coupler may be detachably coupled to the leg portion of the bracket structure. Thus, the coil units may be used with differing types of bracket structure or with differing types of pivoting couplers. Also, the bracket structure or the pivoting couplers can easily be replaced or discarded without the need for new coil units, which the more complex and costly components of the respiration promoting apparatus. Detachable coil units help to safe costs for a patient's treatment and allow for the customization of the respiration promoting apparatus with respect to a patient's needs.

In still a further embodiment of the respiration promoting apparatus of the invention, the first coil unit has a non-flat first coil winding formed from a conductive elongate component, and the second coil unit has a non-flat second coil winding formed from a conductive elongate component. The conductive elongate component is for example a copper or aluminium wire as commonly used for electric coils. However, other electrically conductive materials may be used as well. The first coil winding and the second coil winding can be formed of individual conductive elongate component.

Thereby, in one advantageous variant at least one of the first coil winding and the second coil winding is a convex coil winding. For example, such convex coil winding may result from several spiral-like turns which together form an outwards curved exterior surface of the coil windings. Therefore, the first and the second coil winding can have a convex outer side and a concave inner side. Particularly, the first coil winding and the second coil winding may have an essentially conical shape. Like this, the coil windings can be cone coils or cone coil windings. Also, at least one of the convex coil windings may have a cross section along a winding axis in an essentially parabolic shape, i. e. the parabolic shape can form a portion of a parabola. Further, at least one of the convex coil windings may have a cross section in an essentially spherical shape, which means each of the convex coil windings may have a cross section in an essentially spherical shape. Thereby, the spherical shape can form a portion of a circle.

The outwards curved exterior surface of the coils can be positioned on soft body tissue and with slight, physiologically tolerable pressure on the coils (e.g. created by the coil weight itself). Thus, the coil windings on the outwards curved exterior surface that is impressed into the body can get closer to the target nerve. Any distance reduction between coil wire and target nerve will result into higher effectiveness because electro-magnetic fields are reduced exponentially with increasing distance to the source.

Alternatively, in another advantageous variant at least one of the first coil winding and the second coil winding is designed as essentially cylindrical coil winding. Thus, the coil windings may have a cross section in an essentially cylindrical shape. In this case the rim or edge of an outermost winding can be positioned on the skin and may be pressed to the body just as mentioned for convex shaped coil windings.

Advantageously, both coil windings have the same non-flat shape. Thus, for example each of the coil windings has a cross section in an essentially parabolic shape, in an essentially conical shape or an essentially cylindrical shape.

Advantageously, each of the first and second coil units is bent or curved. More specifically, the forward faces of the first and second coil units can be bent or curved. Like this, the coil units can be designed to extend about or adjacent to a neck of the patient. Particularly, the radius of the curve can be in a range of a human or animal neck.

Furthermore, each or at least one of the first non-flat coil winding and the second non-flat coil winding may have an oval base shape. The base shape can for example be defined by the outermost turn or winding of the coil windings. Generally, all of the spiral-like turns of the coil winding may have an oval shape similar to the oval base shape. However, due to the non-flat nature of the first and second coil winding, turns with a small radius that are close to the coil winding axis may have a shape differing from the turns with larger radii further away from the coil winding axis. The oval base shape may comprise an elliptic shape or a parabolic shape at opposing sides of the winding. By providing coil windings with oval base shapes, oval electro-magnetic fields can be generated. Like this, the electro-magnetic fields can be longitudinally stretched such that, under certain circumstances, the electro-magnetic field can be better provided distant from muscular structures towards the Phrenic nerves. For example, the longitudinally extending electro-magnetic fields facilitate the positioning of the coil units near or adjacent to the sternocleidomastoid muscles for stimulating the Phrenic nerves. Like this, side effects in use of the apparatus can be lowered or prevented. In case the first and second coil windings are cylindrical, an oval base shape may allow for providing a comparably large contact surface by locating a wide curved portion of the oval base shape at the patient such that the coil units can be safely and stably positioned at the patient. Or, the oval base shape may allow to apply a comparably narrow electro-magnetic filed to the patient by positioning the narrow curved portion of the oval shape at the patient and/or to impress the coil unit to a comparably large extent into the skin of a patient. In general, by designing the coil windings with the oval base shape, the electro-magnetic field can be geometrically shaped as desired.

In still a further embodiment of the respiration promoting apparatus of the invention a locking mechanism is provided to block the position and orientation of the first coil unit and the second coil unit relative to each other in a target configuration of the respiration promoting apparatus and the bracket structure, respectively. That means the locking mechanism locks a movement of the leg portions at the hinge portion, a movement of the pivoting coupler relative to the leg portions and a movement of the coil units relative to the pivoting coupler. The locking mechanism can be any suitable mechanism for stopping these movements. For example, the locking mechanism could be realized as a mechanical locking structure or an electrical locking arrangement.

Advantageously, the locking mechanism of the bracket structure is configured to irreversibly block the position and orientation of the first coil unit and the second coil unit relative to each other in the target configuration. Therefore, a distance between the first and second coil units and the orientation of the coil units towards each other, e.g. tilting angle, are fixed. Using one of the forward faces, for example the third forward face on the hinged portion of the bracket structure, as a reference contact point on a patient's body, will automatically place the first coil unit and the second coil unit at the desired contact points for the coil units and production of the electro-magnetic fields.

Further, the respiration promoting apparatus preferably may comprise a control unit configured to coordinatedly provide current to the first coil unit and to the second coil unit, respectively. In one version, the control unit is configured to simultaneously provide current to the first coil unit and to the second coil unit. In another version, the control unit is configured to alternately provide current between the first coil unit and the second coil unit. The control unit may comprise a switch for alternating a current supply between the coil units. Thus, by coordinating each of the current flows in the first and second coil unit the respiration promoting apparatus can coordinatedly stimulate the two Phrenic nerves as required for optimal activation of the diaphragm.

Also, the control unit can comprise a power supply coupled to the first coil winding of the first coil unit and to the second coil winding of the second coil unit. In one variant of the method according to the invention, the control unit controls the power supply to provide a current around 40Hz. Preferably, the control unit is configured such that the power supply provides current through the first coil winding in a circulation direction and through the second coil winding in the same circulation direction. The term "circulation direction" can particularly relate to a clockwise or counter clockwise direction. In such an embodiment, the first and second coil units may be operated in a reverse current mode. Like this, the electromagnetic fields generated by the two coil units can cancel each other in an area between the coil units. Thereby, undesired effects or side effects affecting the patient can be prevented. Also, it can be prevented that the coil units disturb each other in operation.

In one example embodiment the bracket structure or the control unit may include a switch to direct current into either the first coil unit or the second coil unit. Preferably, the switch would switch between coil units after every impulse applied. With such technology, the stimulator could produce a train with double target frequency, and the switch would distribute the pulses alternately between the first and the second coil unit, thus inducing the target train frequency in each coil. An alternating current supply to the first and the second coil unit has two advantages: (1) The pulses in the two coil units are always applied with a time off-set, thus two coil units will not be magnetized at the same time, and the risk of pull-effects or interaction effects between two coil units is mitigated, and (2) the design of the respiration promoting apparatus can be minimized and produced with less costly components, because overall capacity can be reduced because a current to supply one coil unit is smaller than a current to supply two coil units.

Also, the respiration promoting apparatus according to the invention may comprise a first biofeedback sensor coupled to the control unit and a second biofeedback sensor coupled to the control unit. Preferably, the first biofeedback sensor is associated to the first coil unit and the second biofeedback sensor is associated to the second coil unit. The biofeedback sensors are configured to receive an activation feedback signal upon detection of the activation of the Phrenic nerves and/or the diaphragm. Advantageously, the control unit is configured to individually provide current to the first coil unit and to the second coil unit in accordance with the feedback signals received from the first biofeedback sensor and the second biofeedback sensor such that the diaphragm is uniformly activated.

Particularly, in use or method for promoting respiration of a patient the first biofeedback sensor is positioned at the patient such that it provides a first feedback signal about contractility of a first diaphragm hemisphere, and the second biofeedback sensor is positioned at the patient such that it provides a second feedback signal about contractility of a second diaphragm hemisphere. In accordance with the first feedback signal and the second feedback signal, the current is coordinatedly provided through the first coil winding and the second coil winding. By involving the first and second biofeedback sensors, the method allows for automatically stimulating the diaphragm of the patient in a homogeneous manner.

Preferably, the biofeedback sensors comprise at least one electrode configured to be arranged at the patient such that it senses an activity of the respective Phrenic nerve. Such biofeedback sensors can efficiently detect activation of the nerves such that the electro-magnetic field can be calibrated and proper functioning of the stimulation can be monitored.

The control unit may comprise an electro-magnetic field adjustment mechanism that is configured to automatically adjust or vary a field strength or amplitude of the electro-magnetic field generated by the first and second coil units of the respiration promoting apparatus. Particularly, when a predefined threshold of the electro-magnetic field is reached, the field will be reduced and adjusted. Also, the electro-magnetic field adjustment mechanism may automatically stop variation of the field strength of the electro-magnetic field when a satisfying activation feedback is received from the biofeedback sensors. Thus, the control unit allows to efficiently adjust and dimension the electro-magnetic field or its required shape in order to achieve desired depolarization of the membrane of the Phrenic nerves without negative effects on surrounding tissue.

The control unit also may signal the satisfying Phrenic nerve activation feedback as an indicator that the chosen bracket structure configuration equals an optimal configuration and can serve as a target configuration. Therefore, in case the respiration promoting apparatus needs to be rotary internal combustion re-adjusted - for example after taking of the apparatus for other treatments of the patient - the target configuration can easily be reproduced.

The respiration promoting apparatus comprising the coil arrangements and supporting structures described herein are typically provided with biphasic or monophasic currents in the wires. Current directions are typically reversible. The alternating currents induce alternating magnetic fields, which in turn induce alternating electric field components. Those are used to depolarize nerve membranes. An electric field component vertical to the membrane will depolarize the membrane. In embodiments where two adjacent coil units are operated with currents having opposing rotational direction the main electric field component is typically created at the centre between the two adjacent coil windings, in a direction vertical to a virtual axis connecting centres of the two coil windings. Therefore, it is desirable to place this virtual axis parallel to the nerve.

In use of the respiration promoting apparatus for activating the Phrenic nerves, the respiration promoting apparatus generates two peaks in the body areas between the coil windings, wherein advantageously the main electric field peaks are induced above the two Phrenic nerves, and the main electric field component direction is angled more than 20° and less than 160° relative to the Phrenic nerves, in order to induce a potential difference along the nerve membranes.

### Brief Description of the Drawings

The respiration promoting apparatus and the method for promoting respiration of a patient according to the invention are described in more detail herein below by way of exemplary embodiments and with reference to the attached drawings, in which:
- Fig. 1a: shows a first embodiment of a respiration promoting apparatus according to the present invention comprising a first coil unit, a second coil unit and a bracket structure in a narrow configuration;
- Fig. 1b: shows the first embodiment of a respiration promoting apparatus in a wide configuration;
- Fig. 2: shows a second embodiment of a respiration promoting apparatus according to the present invention comprising a reference structure;
- Fig. 3: shows a third embodiment of a respiration promoting apparatus according to the present invention comprising a locking mechanism;
- Fig. 4: shows a fourth embodiment of a respiration promoting apparatus according to the present invention comprising a first coil unit, a second coil unit and a bracket structure with a pivoting couplers for mounting the coil units;
- Fig. 5: shows a schematic view of an arrangement of a first coil winding and a second coil winding of a respiration promoting apparatus according to the present invention;
- Fig. 6a: shows a schematic view of the use of a respiration promoting apparatus according to the present invention comprising a convex first coil winding and convex second coil winding;
- Fig. 6b: shows a schematic view of the use of a respiration promoting apparatus according to the present invention comprising a cylindrical first coil winding and a cylindrical second coil winding, wherein the coil windings are arranged with their cylindrical circumference on the body surface;
- Fig. 6c: shows a schematic view of the use of a respiration promoting apparatus according to the present invention comprising a cylindrical first coil winding and a cylindrical second coil winding, wherein the coil windings are arranged with their outermost coil winding on the body surface;
- Fig. 7a: shows a schematic view of a first coil winding and a second coil winding of the respiration promoting apparatus comprising a circular base shape;
- Fig. 7b: shows a schematic view of a first coil winding and a second coil winding of the respiration promoting apparatus comprising an oval base shape;
- Fig. 7c: shows a schematic view of a first cylindrical coil winding and a second cylindrical coil winding of the respiration promoting apparatus arranged with their cylindrical axes essential perpendicular to a nerve axis;
- Fig. 7d: shows a schematic view of a first cylindrical coil winding and a second cylindrical coil winding of the respiration promoting apparatus arranged with their cylindrical axes essential parallel to a nerve axis;
- Fig. 8: shows a front view of a fifth embodiment of a respiration promoting apparatus according to the present invention;
- Fig. 9: shows a perspective view of the respiration promoting apparatus of Fig. 8;
- Fig. 10: shows a front view of the respiration promoting apparatus of Fig. 8;
- Fig. 11a: shows a portion of a sixth embodiment of respiration promoting apparatus according the invention; and
- Fig. 11b: shows the portion of Fig. 11a, wherein a guide member is removed.

### Description of Embodiments

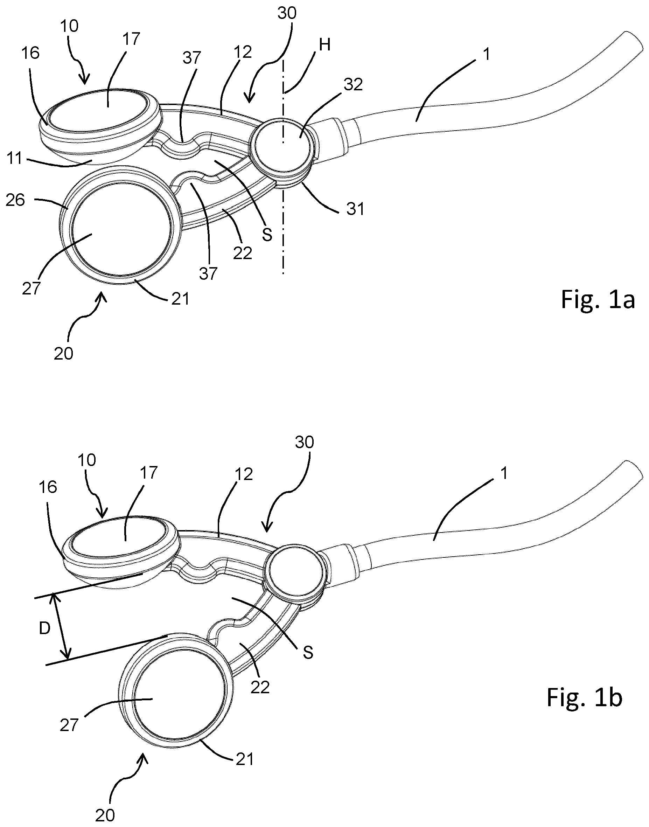

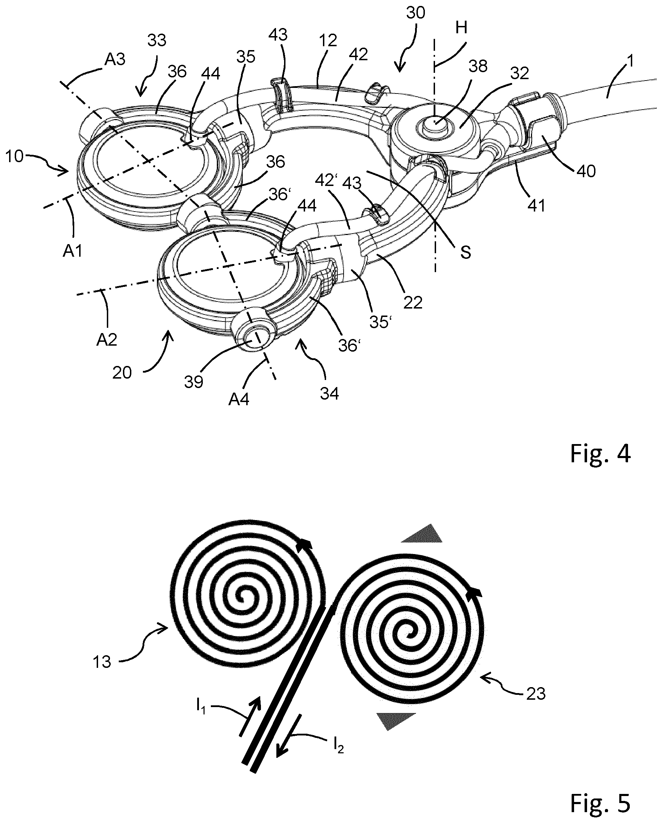

Figures 1 to 4, 8 and 9 show different embodiments of respiration promoting apparatuses according the present invention, which comprise a bracket structure coupled to coil units for positioning the apparatus at a patient and coordinatedly stimulating two Phrenic nerves. Figures 5 to 7 schematically show the concepts and applications of a respiration promoting apparatus of the invention for coordinatedly stimulating the two Phrenic nerves. Further, a method of using a respiration promoting apparatus according to the present invention may use any of the respiration promoting apparatuses and concepts disclosed in Figures 1 to 9. Particularities of the method will be become apparent in context with the illustrated example embodiments. The example embodiments of a respiration promoting apparatus shown in Figures 1 to 4, 8 and 9 may take advantage of the concepts and applications as disclosed in Figures 5 to 7, but also may use other, more conventional coil arrangements.

In the following description certain terms are used for reasons of convenience and are not intended to limit the invention. The terms "right", "left", "forward", "backward", etc. refer to positions relative to a patient unless otherwise indicated the description. The terminology comprises the explicitly mentioned terms as well as their derivations and terms with a similar meaning. Also, spatially relative terms, such as "beneath", "below", "larger", "above", "upper", "smaller", "proximal", "distal", and the like,
may be used to describe one element's or feature's relationship to another element or feature as illustrated in the figures or indicated in the description.

To avoid repetition in the figures and the descriptions of the various aspects and illustrative embodiments, it should be understood that many features are common to many aspects and embodiments. Omission of an aspect from a description or figure does not imply that the aspect is missing in embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part or component are provided with reference signs it is referred to other drawings showing the same part or component. Like numbers in two or more figures represent the same or similar elements or elements with the same functionality.

Figures 1a and 1b illustrate a first embodiment of a respiration promoting apparatus to coordinately stimulate two Phrenic nerves (not shown) of a patient for activating a diaphragm of the patient. The respiration promoting apparatus comprises a first coil unit 10 having a first forward face 11 configured to be positioned at the patient to stimulate a first Phrenic nerve of the patient and a second coil unit 20 having a second forward face 21 configured to be positioned at the patient to stimulate a second Phrenic nerve of the patient.

Each of the coil units 10, 20 comprises a housing 16, 26 for accommodating the associated coil winding 13, 23 and providing contact surfaces to be positioned at the patient. The first coil unit 10 has a first housing 16 encasing a first coil winding 13 and the second coil unit 20 has a second housing 26 encasing a second coil winding 23. By such housings 16, 26 the coil windings 13, 23 of the coil units 10, 20 can be encased and protected. Also, the housing 16, 26 with the coil winding 13, 23 safely positioned and precisely oriented therein allows for assuring correct positioning and orienting of the coil winding relative to the patient and relative to each other. Further, the housings 16, 26 can provide suitable surfaces for the forward faces of the coil units 10, 20, for example to be positioned at the patient. Thus, a surface of the first housing 16 serves as the first forward face 11 of the first coil unit 10 and a surface of the second housing 26 serves as the second forward face 21 of the first coil unit 20. Furthermore, the housings 16, 26 may carry a shielding as will be explained in more detail below.

The respiration promoting apparatus further comprises a bracket structure 30 coupled to the first coil unit 10 and the second coil unit 20. Advantageously, the coil units 10, 20 can be detachably coupled to the bracket structure 30. In this case, the bracket structure 30 can easily be replaced, e.g. disposed, in case needed.

The bracket structure 30 is releasably connected to a current supply cable 1 for coordinatedly providing current to the first and the second coil units 10, 20 for generating electro-magnetic fields such that the first Phrenic nerve 62 and the second Phrenic nerve 62 of the patient are stimulated and a diaphragm of the patient is homogeneously activated. For generating the electro-magnetic fields, each of the first and the second coil units 10, 20 comprises the non-flat coil winding 13, 23 as described with respect to Figures 5 to 7. However, conventional flat coil windings may be used as well without departing from the present invention or foregoing any of the benefits of the invention. The elongate component can for example be a metal wire such as a copper wire or the like.

The coil windings 13, 23 can be positioned and fixed within the housings 16, 26 of the coil units 10, 20 such that a side of the coil winding 13, 23 that is determined to face towards the patient is oriented towards the housing surface providing the forward face 11, 21 of the respective coil unit 10, 20. In case of non-flat coil windings 13, 23, as for example a convex coil winding as further described below, the housing 16, 26 may have a similar three-dimensional shape as the coil windings 13, 23. Thus, the orientation of the non-flat coil winding 13, 23 towards a patient can be visually monitored in a simple manner. The housings 16, 26 shown in Figures 1 to 4 have a flat surface representing backward faces 17, 27 of the coil units 10, 20, and a bulged surface representing the forward faces 11 and 21. Further, circumferential sides of the housing 16, 26 connect the backward facing surface and the forward facing surface.

The bracket structure 30 is adjusted such that positions and orientations of the first coil unit 10 and of the second coil unit 20 are adapted to the specific needs of a patient and for generating an electro-magnetic field targeted for stimulating the Phrenic nerves of the patient. The bracket structure 30 has a third forward face 31 configured to be positioned at the body of the patient. The respiration promoting apparatus is configured to be arranged at a defined location of the patient when the first forward face 11, the second forward face 21 and the third forward face 31 are positioned at the patient in a chosen configuration of the bracket structure 30 suitable for the specifics of the treatment of the patient. Thus, the positioning and adjustment of the three forward faces 11, 21, 31 define a target configuration of the respiration promoting apparatus.

In use the respiration promoting apparatus is arranged at a defined location of the patient such that the first forward face 11, the second forward face 21 and a third forward face 31 are in contact with the patient. When being positioned at the body, the three forward faces serve as three support points and determine defined contact points on the body. Like this, the respiration promoting apparatus can be stably and precisely positioned with respect to the patient's Phrenic nerves, and at the same time provides access to important areas of the patient's neck. For example, tracheotomy may conveniently be possible while stimulating the Phrenic nerves. The electro-magnetic field generated by the first and the second coil units 10, 20 can be precisely targeted to their respective target Phrenic nerve 62 without implying co-stimulation of other body tissue.

In the embodiment shown in Figures 1a and 1b, the bracket structure 30 comprises a first leg portion 12, a second leg portion 22 and a hinge portion 32 connecting the first leg portion 12 and the second leg portion 23 with each other. In this embodiment the first leg portion 12 and the second leg portion 22 are realized as flat rod elements having a curved shape. The first coil unit 10 is releasably coupled to one end of the first leg portion 12 and the second coil unit 20 is releasably coupled to one end of the second leg portion 22. The hinge portion 32 is arranged at an opposing end of the first and second leg portions 12, 22 and flexibly couples the first and second leg portions 12, 22 at their opposing ends.

Further, the rod elements serving as the first and second leg portions 12, 22 each comprise a bulge 39 that extends from a middle section of the rod elements. The bulges 39 may serve for manually handling and adjusting the bracket structure 30. Also, the rod elements may each comprise a fixation arrangement for releasably holding a current line that runs along the leg portion and connects the current supply cable 1 at one end of the leg portions to the coil units 10, 20 at the other end of the leg portions 12, 22. An example for a fixation arrangement is shown in Figure 4.

In the embodiment of Figures 1a and 1b, the hinge portion 32 is configured as a pivot bearing such that the first and second leg portions can be pivoted around a common articulation axis H of the bearing that runs through the centre of the hinge portion 32. Thus, the leg portions 12, 22 can be swivelled around the axis H in a common two-dimensional plane. Alternatively, the hinge portion 32 could provide more than one articulation axis for adjusting the leg portions three-dimensionally. In this case, the hinge portion 32 could for example be configured as a ball bearing.

The hinge portion 32 allows for adjusting a distance D between the first coil unit 10 and the second coil unit 20 to adapt the position and orientation of the first and second coil units 10, 20 according a desired target configuration of the bracket structure 30 depending on the requirements for use of the respiration promoting apparatus. Figure 1a shows the respiration promoting apparatus in a narrow configuration of the bracket structure 30 wherein the first coil unit 10 and the second coil unit 20 are in a short distance D from each other. Figure 1b shows the respiration promoting apparatus in a wide configuration of the bracket structure with a larger distance D between the first coil unit 10 and the second coil unit 20.

The curved shape of the first leg portion 12 and the second leg portion 22 guaranties that in any pivoting position of the first leg portion 12 and the second leg portion 22 relative to each other a free space S is provided between the hinge portion 32 and the first leg portion 12 and the second leg portion 22. However, the free space S of the narrow configuration of the bracket structure provides a smaller free area between the leg portions 12, 22 than the wide configuration. In any case, the free space provides ample access to a front area of the neck of a patient that is commonly important for treatment of the patient such as a tracheotomy. The leg portions 12, 22 shown in Figures 1a and 1b are curved in one geometrical plane. However, additionally they could be curved in a second geometrical plane, for example perpendicular to the one plane. Thus, they could create an arch providing additional free space and access to the patient for example from the sides of the neck.

Advantageously, the respiration promoting apparatus rests on the body surface of the patient only with the three forward surfaces 11, 21, 31 located at the first coil unit 10, the second coil unit 20 and at the bracket structure 30. Thus, the three forward surfaces 11, 21, 31 provide support points in a triangular geometry. This enables stable and well-defined positioning at the patient independent of an individual surface landscape of the patient's body while space constraints can be overcome and the respiration promoting apparatus can be carried by the patient conveniently and pain-free. Further, the geometry of the bracket structure determines a target configuration of the respiration promoting apparatus and determines a defined location and orientation of the coil units generating the electro-magnetic fields for activating the diaphragm. This results in efficient stimulation of both Phrenic nerves and avoids co-stimulation effects of tissue in the vicinity of the two Phrenic nerves, particularly in between the two Phrenic nerves where the field could interfere with other medical treatment.

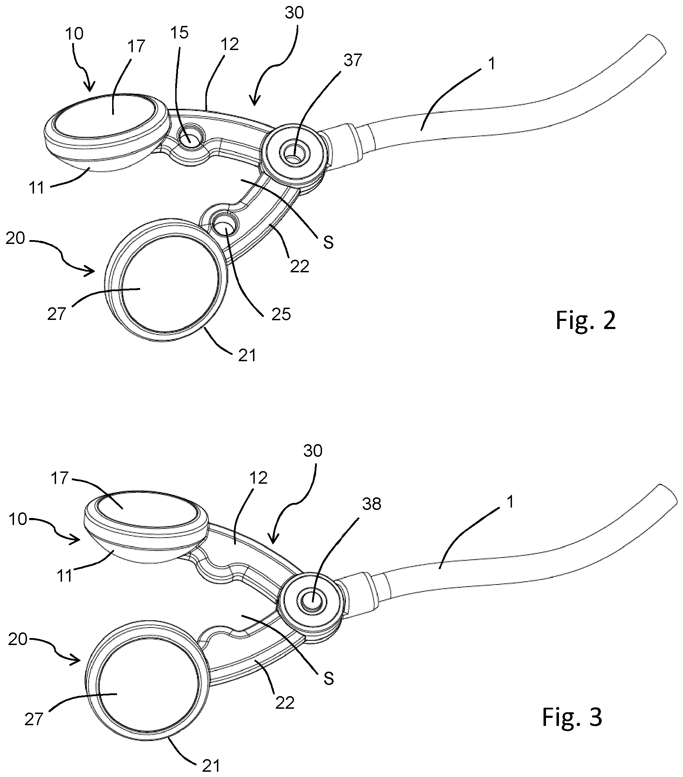

The embodiments of the respiration promoting apparatus according to the invention as shown in Figures 2 to 4 are generally configured analogue the embodiment of Figure 1 but include additional or alternative features to amend the use of the respiration promoting apparatus as described in the following.

In a second embodiment illustrated in Figures 2, the bracket structure 30 comprises a through-hole 15 in the first leg portion 12, a through-hole 25 in the second leg portion 22 and a through-hole 37 in the hinge portion 32. The through-holes 15, 25, 37 may serve as a reference structure configured to repeatedly locate the respiration promoting apparatus at the defined location of the patient's body. The through-holes 15, 25, 37 allow to align the bracket structure with a specific portion of the body such as at a landmark thereof. By having the reference structure and for example using the natural landmarks of the body, the respiration promoting apparatus can conveniently be repositioned at the body such that it can be assured that the first and second coil units 10, 20 are properly positioned and oriented to stimulate the first and second Phrenic nerves.

Further, the through-holes 15, 25, 37 can be designed to provide markers to the patient's body there through, when the respiration promoting apparatus is in a desired target configuration. The markers can, for example, be provided by a pen or similar item applying a visual sign on the patient's body. Thus, the markers on the patient's body assist to properly position and orient the first and second coil units 10, 20.

The natural body landmarks and/or the markers also may assist in re-adjusting a bracket structure 30 to an already known suitable configuration. The position and orientation of the coil units 10, 20 can be re-adapted by moving the leg portions 12, 22 to align the landmarks and/or markers with the through-holes 15, 25, 37. Thus, in use of the respiration promoting apparatus the bracket structure can easily be exchanged.

In a third embodiment illustrated in Figure 3, the bracket structure 30 of the respiration promoting apparatus according to the present invention comprises a locking mechanism to block the position and orientation of the first coil unit 10 and the second coil unit 20 relative to each other in the target configuration of the bracket structure 30. The locking mechanism can for example be provided by a mechanical mechanism or an electrical arrangement. In the present embodiment, the locking mechanism is realized by a locking button 38, which blocks rotation of the first leg portion 12 and the second leg portion 22 in the hinge portion 32 of the bracket structure 30. The locking button 38 can block the movement of the leg portions 12, 22 for example by providing a form fit within the hinge portion 32 in a pushed in or turned position. Alternatively or additionally, the locking button 38 could block the leg portions 12, 22 by frictional locking. The locking mechanism can prevent changes of the configuration of the bracket structure 30 after the target configuration has been adjusted, which guarantees adequate stimulation of the Phrenic nerves by the respiration promoting apparatus. Thus, the respiration promoting apparatus can be removed from the patient and replaced at a later point in time without the need of recalibration of the coil units 10, 20.

The locking mechanism of the bracket structure 30 can be configured to irreversibly block the position and orientation of the first coil unit 10 and the second coil unit 20 relative to each other in the target configuration of the bracket structure 30. By irreversibly blocking the bracket structure the target configuration cannot accidentally be changed whereby a new adjustment of the respiration promoting apparatus would become necessary.

In a fourth embodiment of the respiration promoting apparatus according to the invention shown in Figure 4, the coil units 10, 20 are coupled to the bracket structure 30 using U-like shaped pivoting couplers, which are detachably mounted to the bracket structure 30. The U-shape is realized by a stem portion 35 and two symmetrically curved branch portions 36 of the pivoting couplers. As shown in Figure 4, a first pivoting coupler 33 has a stem portion 35 and two branch portions 36, and a second pivoting coupler 34 has a stem portion 35' and two branch portions 36'. The stem portion 35 of the first pivoting coupler 33 is mounted to an end of the first leg portion 12 and the stem portion 35' of the second pivoting coupler 34 is mounted to an end of the second leg portion 22. The pivoting couplers 33 and 34 basically are arranged in extension of the leg portions at an end thereof. The first coil unit 10 is mounted between the two branch portions 36 of the first pivoting coupler 33 and the second coil unit 20 is mounted between the two branch portions 36' of the second pivoting coupler 34.

The current supply cable 1 is releasably held in a clip mounting 40 arranged at an extension plate 41 extending from the hinge portion 32. The supply cable 1 feeds two current lines 42, 42', which run along the bracket structure 30 and provide current pulses to the first coil unit 10 and the second coil unit 20. The current lines are detachably fixed to the leg portions by fixation fingers 43. The current lines 42, 42' are connected to the coil units by common connectors 44. The supply cable 1 and the current lines 42, 42' can be detached from the bracket structure 30 and from the coil units 10, 20. Thus, they can be used with a different bracket structure and different coil units and the respiration promoting apparatus can easily be modified for different use situations. For example, the bracket structures can be embodied as disposables associated to a specific patient and to be replaced after treatment of the specific patient.

The first pivoting coupler 33 is pivotably mounted to the first leg portion 12 such that the first pivoting coupler 33 can be pivoted relative to the first leg portion 24 about an axis A1 of the first leg portion 12. Respectively, the second pivoting coupler 34 is pivotably mounted to the second leg portion 22 such that the second pivoting coupler 34 can be pivoted relative to the second leg portion 22 about an axis A2 of the second leg portion 22. The leg portion axes A1, A2 are basically aligned with the longitudinal direction of the end portions of the curved leg portion elements and the longitudinal axis of the stem portion 35 of the pivoting couplers. Thus, depending on an adjustment angle of the first leg portion 12 and the second leg portion 22 relative to each other at the hinge portion the leg portion axis A1 and the leg portion axis A2 can enclose different angles. However, pivoting the first coil unit 10 around axis A1 and pivoting the second coil unit 20 around axis A2 can cause the coil units 10, 20 to face more towards or more away from each other in most hinge portion adjustment angles. This can for example result in an increase or decrease of an overlap of the electro-magnetic fields generated by the coil units 10, 20, respectively, and facilitate the alignment of the electro-magnetic fields with the position of the Phrenic nerves.

The first and second coil units 10, 20 are also pivotably mounted between the branch portions 36 such that the first coil unit 10 can be pivoted relative to the first pivoting coupler 33 about an axis A3 and the second coil unit 20 can be pivoted relative to the second pivoting coupler 34 about an axis A4. The axes A3 and A4 basically connect the ends of the branch portions 36, 36' of the respective pivoting coupler.

In summary, the axes A1, A2 along the longitudinal axis of the stem portion 35 define first articulation axes of the pivoting couplers, and the axes A3, A4 crossing the ends of the branch portions 36, 36' within each pivoting coupler define second articulation axes of the pivoting couplers. The first and second articulation axes are essentially perpendicular to each other. Therefore, the coil units 10, 20 can be adjusted in any three-dimensional direction and accordingly an electro-magnetic field generated by the coil units 10, 20 can be provided in any area required for an optimized stimulation field targeting both of the Phrenic nerves. The angle adjustment of the first and the second coil units 10, 20 for optimized orientation of the coil units 10, 20 can be performed individually according to specific needs of the patient and for generating the targeted electro-magnetic field for stimulating the Phrenic nerves 62 of the patient. Thus, negative side effects during the treatment can be avoided and contact points convenient for the patient can be found.

The coil units 10, 20 are shielded to reduce or avoid generating an electro-magnetic field in other areas than needed for stimulation of the Phrenic nerves. Therefore, at least the backward face 17 of the first coil unit 10 and the backward face 27 of the second coil unit 20 are shielded. By shielding the backward faces 11, 21 of the coil units 10, 20 and the coil windings, respectively, it can be prevented that persons or other medical devices are exposed to an electromagnetic field backwardly diverging from the coil units 10, 20. The shielding can be provided at the housings 16, 26 of the coil units 10, 20. Shielding material can be attached to the housing 16, 26 or the housing 16, 26 itself may be made of material with shielding properties at surfaces requiring shielding. Accordingly, at least the backward housing surface has a shielding to prevent field components away from the patient.

Preferably, also lateral faces of the first coil unit 10 and the second coil unit 20 are shielded. In the embodiments of coil units 10, 20 presented, their lateral faces match with the circumferential side of the housing 16, 26, which connects the backward surface and the forward surface of the housing 16, 26. As a result, the coil units 10, 20 generate an electro-magnetic field only in forward direction areas towards a patient. Each of the coil units 10, 20 neither produces field components in areas backwards nor sideward towards the other of the two coil units 10, 20. Therefore, the electro-magnetic fields of the two coil units 10, 20 only may overlap in their forward direction to create a targeted field for stimulating the Phrenic nerves 62, which helps to control the overall electro-magnetic field provided by the respiration promoting apparatus.

For example, the backward and/or lateral faces of the coil units can be shielded by a passive shield such as a u-Metal, a SCM, a passive coil winding, or the like. More advanced, the respiration promoting apparatus may comprise an active shielding member configured to shield the backward faces of the first coil unit and the second coil unit. The active shielding member can comprise coil windings configured to cancel out field and/or change of field direction. For example, the shielding member can comprise a coil winding with a synchronized current in opposing direction with respect to the first or second coil windings 13, 23 of the coil units 10, 20. The active shielding members may conveniently be positioned within the housings 16, 26 of the coil units 10, 20.

Figures 5 to 8 illustrate concepts of a respiration promoting apparatus to coordinatedly stimulate two Phrenic nerves of a patient for activating a diaphragm of the patient according to the present invention. Generally, the respiration promoting apparatus can be designed for example like any of the embodiments of a respiration promoting apparatus shown in Figures 1 to 4. Therefore, like reference numbers in the figures represent the same or similar elements, or elements with same or similar function. Again, the respiration promoting apparatus comprises a first coil unit 10 configured to be positioned at the patient to stimulate a first Phrenic nerve of the patient, and a second coil unit 20 configured to be positioned at the patient to stimulate a second Phrenic nerve of the patient.

The first coil unit 10 has a non-flat first coil winding 13 formed from a conductive elongate component and the second coil unit 20 has a non-flat second coil winding 23 also formed from a conductive elongate component. Each of the coil windings is formed by a plurality of spiral-like or helically wound turns of the conductive elongate component around a winding axis C of the coil winding. Successive turns decrease in radius starting from an outermost turn towards an innermost turn close to the winding axis.

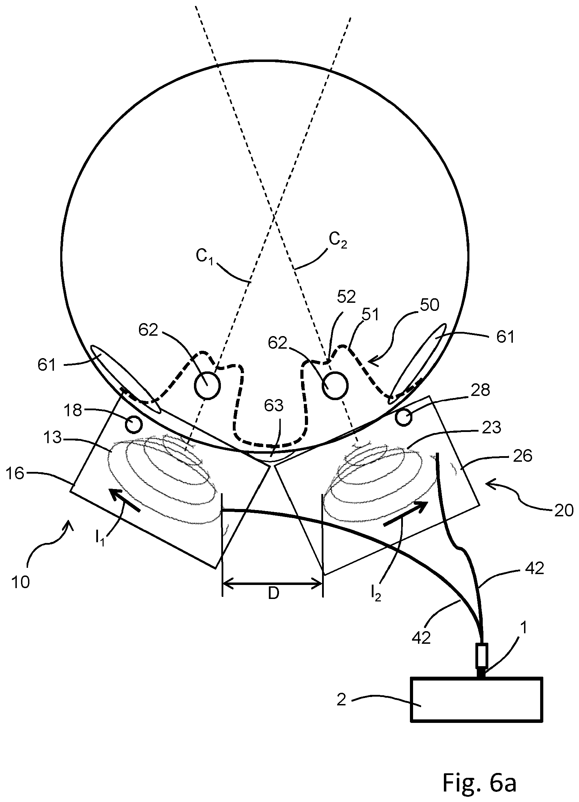

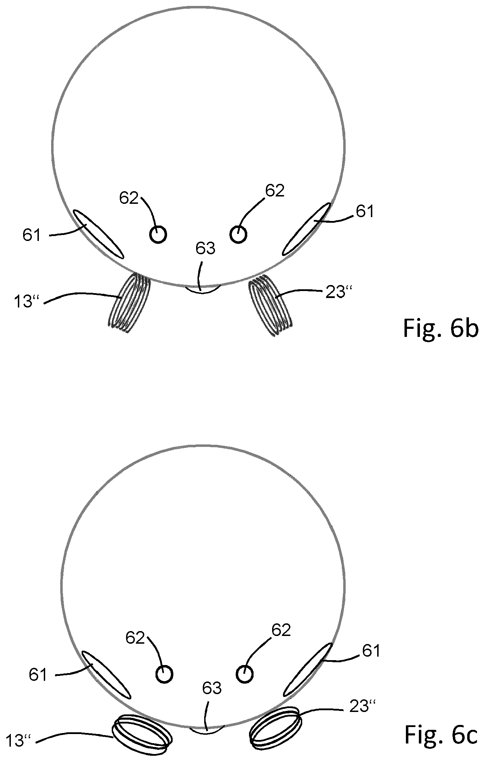

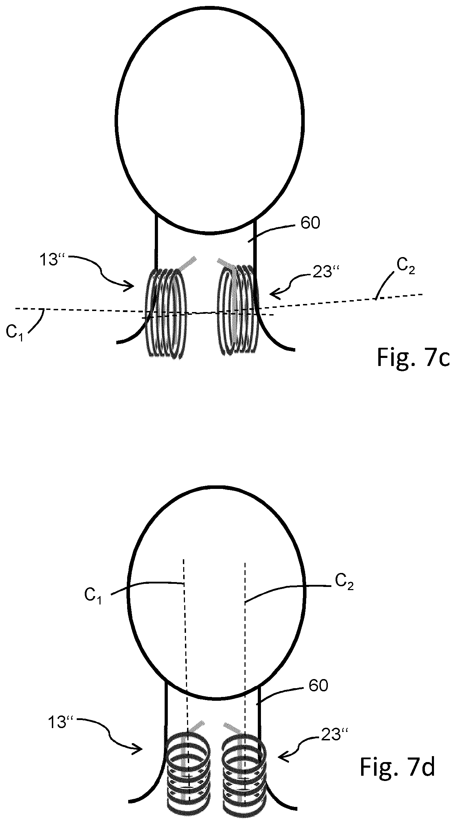

Furthermore, at least some of the turns are shifted along the winding axis to realize a non-flat shape of the first and second coil winding. Therefore, the first and the second coil windings 13, 23 are designed as three-dimensional bodies. Figures 6a, 7a, 7b and 12b show examples, wherein the coil windings have a convex shape, particularly an essentially conical shape. Figures 6b, 6c, 7c and 7d show examples, wherein the coil windings have a cylindrical shape.

As described before, the non-flat coil windings 13, 23 can be accommodated in housings 16, 26 of the coil units 10, 20. In case of concave coil windings, an outermost turn of the coil winding 13, 23 having the largest diameter is located towards the backward face of the housing 16, 26. The innermost turn of the coil winding 13, 23 having the smallest diameter is located towards the forward face of the housing 16, 26. Thus, the coil windings 13, 23 are positioned in the housings 16, 26 with their base side facing the backward facing surface of the housing 16, 26. Correspondingly, their convex outward facing side is directed towards the forward facing surface of the housing. Thus, the base side of the coil windings 13, 23 corresponds to the backward faces 17, 27 of the coil units 10, 20 and the outer side of the convex area of the coil windings 13, 23 corresponds to the forward faces 11, 12 of the coil units 10, 20. By having the convex side of the coil windings directed towards the forward faces 11, 21, it is achieved that in use of the respiration promoting apparatus, the convex sides of the coil windings 13, 23 are directed to the patient. Therefore, such housing 16, 26 assures precise positioning of the coil units 10, 20 with the coil winding 13, 23 at the patient.

In Figure 5 the first non-flat coil winding 13 of the coil unit 10 and the second non-flat coil winding 23 of the coil unit 20 are shown in close vicinity to illustrate their respective winding direction and current flow direction. However, as described before when using the respiration promoting apparatus the first coil unit 10 and the second coil unit 20 preferably are arranged in a distance D from each other. In the example shown in Figure 5 the winding direction of the first coil winding 13 is anti-clockwise and the winding direction of the second coil winding 23 is clockwise. A power supply 2 supplies a current I₁ to the first coil winding 13 and a current I₂ to the second coil winding 23, for example via a current supply cable 1 and current lines 42 as shown before. The currents I₁ and I₂ are supplied such that the current flows of the currents I₁ and I₂ run in opposing direction through the first and second coil windings 13, 23. Therefore, in an area between the coil unit 10 and the coil unit 20 the electro-magnetic fields resulting from the first coil winding 13 and the second coil winding 23 weaken or even cancel out
each other. In an alternative example, the winding direction of the first coil winding 13 and the winding direction of the second coil winding 23 can be identical.

Figures 6a to 6c show a schematic view of a respiration promoting apparatus according to the present invention in use at a neck 60 of a patient seen from the top of the neck. In favour of a better comprehensibility, some of the components of the respiration promoting apparatus are not illustrated in the figure. As mentioned before, the first coil unit 10 and the second coil unit 20 each comprise a conical coil winding 13 and 23. The first coil unit with the first coil winding 13 is placed at an anterior border of a right sternocleidomastoid muscle 61, and the second coil unit with the second coil winding 23 is placed at an anterior border of a left sternocleidomastoid muscle 61, wherein the larynx 63 lies in between the two coil windings 13, 23. The first and the second coil windings 13, 23 are spaced apart in distance D from each other. The distance D can vary according to varying physiologies of different patients and different treatments provided for the patient. A first winding axis C1 of the first coil winding 13 and a second winding axis C2 of the second coil winding 23 converge in direction towards the patient. Preferably, the first and the second winding axis C1 and C2 at least approximately intersect with the respective Phrenic nerve associated to the coil windings 13, 23. Preferably, the first and the second winding axis C1 and C2 lie in a common plane, which facilitates optimizing the electrical field exerted on the Phrenic nerves.

Alternatively to the convex shape of the coil windings 13 and 23 as shown in Figure 6a, the coil winding could be realized as cylindrically shape coil windings 13" and 23" as shown for example in Figures 6b and 6c described in more detail below. Advantageously, a respiration promoting apparatus comprising a bracket structure as discussed above is used for adjusting the positions and orientations of the coil units 10, 20. Like this, the coil windings 13, 23 can reliably and repeatedly positioned at the patient by taking advantage of support points provided by the coil units or their housings respectively. The term, "positioned at" as used herein can relate to being located at a patient's body by contacting it. For example, the three forward faces 11, 21, 31 of the stimulation device can be positioned at the body by lying on it. When being positioned at the body, the three forward faces 11, 21, 31 can form three support points and determine defined contact points on the body. Like this, the respiration promoting apparatus can be stably and precisely positioned with respect to the patient's Phrenic nerves. Further, by choosing a specific target configuration of the bracket structure 30 and specific contact points on the patient's body a defined location of the respiration promoting apparatus with respect to the patient is determined. The defined location is defined in that the first forward face 11, the second forward face 21 and the third forward face 31 provided at the bracket structure 30 are in contact with the patient at a position optimized for stimulating the Phrenic nerves 62.

In case that coil unit positioning without a bracket structure as discussed before is chosen, only two support points may be available corresponding to the first forward face of the first coil unit and the second forward face of the second coil unit. However, positions and orientations of the coil units may for example be adjusted manually. Alternatively, a suitable support structure may be provided configured to hold the first and the second coil units in a pre-set or adjustable position and orientation towards each other, which are suitable for generating the targeted electro-magnetic field of the respiration promoting apparatus.

The power supply 2 provides a current flow in the coil windings 13, 23 for example via a common current supply cable 1 and two current lines 42 running to each of the coil windings 13, 23. The power supply 2 advantageously is connected to or may include a control unit configured to coordinatedly provide a current flow through to the first and second coil windings 13, 23. Preferably, the control unit is configured such that current is provided simultaneously to the first and the second coil windings 13, 23 according to a circulation direction for each of the coil winding required to generate a target electro-magnetic field suitable to stimulate the two Phrenic nerves without impacting surrounding tissue.

In the example shown in Figure 6a the winding directions of the two coil windings 13, 23 are identical. Therefore, the current direction is controlled by the control unit such that a flow direction of current I₁ in the first coil winding 13 is opposite to a flow direction of current I₂ in the second coil winding. Each of the coil windings 13, 23 generates an electro-magnetic field 50 targeted towards their associated Phrenic nerve 62. The two electro-magnetic fields 50 decrease toward an area in between the two coil windings 13, 23, but any remaining overlap of the fields would result in mutual erasure of the fields and the resulting overall electro-magnetic field strength would be zero or near zero in this area. Consequently, for example the area around the larynx would not be affected by any electro-magnetic field generated by the respiration promoting apparatus. When using a respiration promoting apparatus having a bracket structure 30 as discussed above, this area also is kept clear of any components of the apparatus and is freely accessible for the treatment of the patient.

Further, in the shown embodiment the respiration promoting apparatus comprises a first biofeedback sensor 18 and a second biofeedback sensor 28. The biofeedback sensors 18 and 28 are coupled to the control unit and the power supply, respectively. When the coil units 10, 20 are positioned at the patient, the biofeedback sensors 18 and 28 are oriented such that they can receive feedback signals from the Phrenic nerves and the diaphragm, respectively, when current is provided through the first and second coil windings 13, 23. For example, the first biofeedback sensor is positioned at the patient such that it provides a first feedback signal about contractility of a first diaphragm hemisphere, and the second biofeedback sensor is positioned at the patient such that it provides a second feedback signal about contractility of a second diaphragm hemisphere. E.g. the biofeedback sensors can comprise electrodes to be positioned at or near the diaphragm of the patient. Thus, the first coil winding 10 and the second coil winding 20 can be controlled coordinatedly in accordance with the first feedback signal and the second feedback signal and the power supply can individually provide a current to each of the coil windings 13, 23. For example, a current strength or a pulse frequency can be changed in response to the feedback signals such that the first and second Phrenic nerves are stimulated and a diaphragm of the patient is homogeneously activated.

Figures 6b and 6c schematically illustrate the use of an embodiment of a respiration promoting apparatus according to the invention having non-flat coil windings with a cylindrical shape. In Figure 6b, a cylindrical first coil winding 13" and a cylindrical second coil winding 23" are arranged with their circumferential surface on the body surface with their winding axes arranged essentially perpendicular to an axis of the neck and the axes of the Phrenic nerves 62. In Figure 6c, a cylindrical first coil winding 13" and a cylindrical second coil winding 23" are arranged with their circumferential surface on the body surface with their winding axes arranged essentially parallel to an axis of the neck and the axes of the Phrenic nerves 62. However, the first and the second coil windings 13" and 23" are preferably arranged in coil units coupled to a bracket structure as discussed for Figures 1 to 4 for positioning the apparatus at a patient and coordinatedly stimulating the two Phrenic nerves. Thus, position and orientation of the coil windings 13" and 23" can easily be fine-tuned relative to specific requirements of an individual patient. The first and the second coil windings 13" and 23" can for example be arranged in the housings 16 and 26 of the bracket structure 30 such that their circumferential surface is oriented towards the forward faces 11, 21 and the backward faces 17, 27, respectively. Adjusting the first and the second coil windings 13" and 23" by moving their coil units around the articulation axes of the bracket structure will optimize the electro-magnetic field distribution for stimulating the Phrenic nerves.

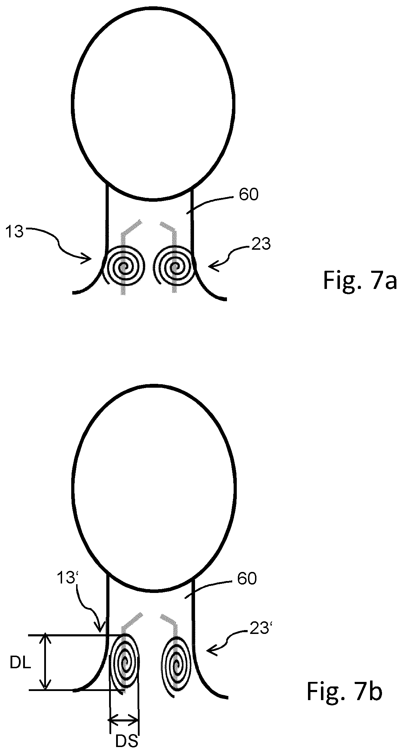

Figure 7a shows a schematic example of coil windings 13, 23 as presented in Figure 6 and their placement at the neck 60 of the patient. As illustrated, the coil windings basically have circular shaped turns. They are essentially symmetric around their winding axis, although of course the turns are linked spiral-like to form a winding. Their base shape as defined by their turn with largest diameter can be described as being circular. According to their shape, such coil windings generate an electro-magnetic field that is symmetric around a centre of the field and turning the coil unit around its winding axis will not change the field distribution in the areas around the coil unit.

In contrast to that, Figure 7b shows a schematic example of coil windings 13', 23' comprising an oval shape and their orientation at the neck 60 of the patient. The turns of the coil windings 13', 23' are basically oval shaped. They are essentially symmetric to a plan including the winding axis. Their base shape as defined by their outermost turn is oval. Consequently, the outermost turn will have a large diameter DL in a first direction and a smaller diameter DS in a second direction perpendicular to the first direction.

The oval shape of the coil windings 13', 23' generates an oval shaped distribution of the electro-magnetic field produced by the coil units. A coil winding with an oval base shape produces an electro-magnetic field that is squeezed at opposing sides, when compared with the field produced by a coil winding of circular shape, if the large diameter DL of the oval shaped coil winding is equal to the diameter of the circular shaped coil winding. Consequently, the distribution of the electro-magnetic field of the oval shaped coil winding reaches less far in direction of the smaller diameter DS than in direction of the large diameter DL. Therefore, the electro-magnetic field resulting from the oval shaped coil winding 13', 23' can be more distant from critical tissue surrounding the Phrenic nerves while still reaching the Phrenic nerves. Also, this allows positioning the coil units with oval shaped coil windings closer to such tissue than coil units with circular shaped coil winding without affecting such tissue. Advantageously, the two coil units 10, 20 are positioned such that their large diameters DL are parallel or nearly parallel to each other. For example the first coil unit 10 having an oval shaped coil winding 13' and the second coil unit 20 having an oval shaped coil winding 23' can be placed closer to the sternocleidomastoid muscles, which may improve stimulation of the Phrenic nerves without impacting the muscles and may provide more space between the first and the second coil unit 10 and 20.

By providing coil windings 13', 23' with oval base shapes, oval electro-magnetic fields can be generated. Like this, the electro-magnetic fields can be longitudinally stretched such that, under certain circumstances, the electro-magnetic field can be better provided distant from muscular structures towards the Phrenic nerves. For example, the longitudinally extending electro-magnetic fields facilitate the positioning of the coil units near or adjacent to the sternocleidomastoid muscles for stimulating the Phrenic nerves. Like this, side effects in use of the apparatus can be lowered or prevented.

Figures 7c and 7d shows a schematic examples of cylindrically shaped coil windings 13", 23" as presented in Figures 6b and 6c and their placement at the neck 60 of the patient. As illustrated, the coil windings basically have circular or oval shaped turns with identical diameters, and they are essentially symmetric along their winding axes C₁ and C₂. In Figure 7c, the first and the second coil windings 13" and 23" are arranged with their circumferential surface towards the neck 60 of the patient. The winding axis C₁ of the cylindrical coil winding 13" and the winding axis C₂ of the cylindrical coil winding 23" are approximately perpendicular to an axis of the neck 60. In Figure 7d, the first and the second coil windings 13" and 23" also are arranged with their circumferential surface towards the neck 60 of the patient. But their winding axes C₁ and C₂ are approximately parallel to an axis of the neck 60. However, the angles between the winding axes C₁ and C₂ can be adapted and also the angle between the winding axes C₁ and C₂ and the neck axis can be adapted for generating a targeted electro-magnetic field.

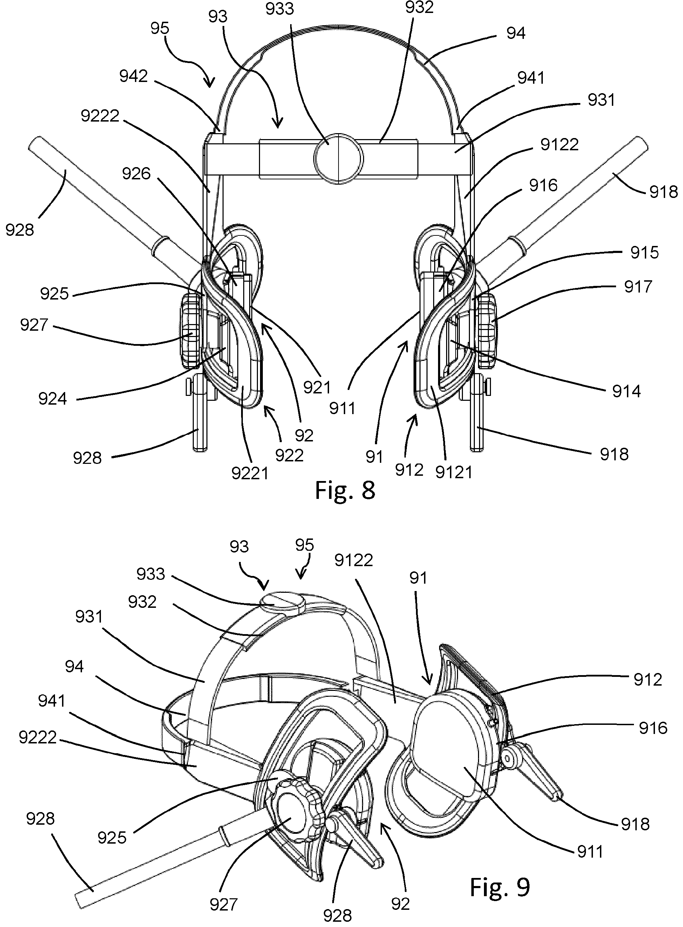

Fig. 8 shows a fifth embodiment of a respiration promoting apparatus according to the invention, which is generally earphone-like shaped. The fifth respiration promoting apparatus comprises a first coil unit 91, a second coil unit 92 and a bracket structure 95 to which the first and second coil units 91, 92 are coupled as described in more detail below.

The bracket structure 95 has an overhead arch 94 bent about 180°, a first frame portion 912 and a second frame portion 922. The overhead arch 94 forms a righthand first tangential end 941 and a left-hand second tangential end 942. The first and second frame portions 912, 922 are essentially mirror symmetrically designed. The first frame portion 911 consist of a bent first rail section 9121 as first neck position guide section and a first height adjusting neck section 9122. Likewise, the second frame portion 921 consists of a bent second rail section 9221 as neck position guide section and a second height adjusting neck section 9222. The first and second height adjusting neck sections 9122, 9222 are essentially rod shaped and extend upwardly from the first and second rail sections 9121, 9221, respectively. At its top end, the first height adjusting neck section 9122 is connected to the first tangential end 941 of the overhead arch 94 and, at its top end, the second height adjusting neck portion 9222 is connected to the second tangential end 942 of the overhead arch 94.

In addition to the overhead arch 94, the first and second height adjusting neck sections 9122, 9222 are interconnected by a forehead support 93. The forehead support 93 comprises two bands 931 which are centrally connected via a sizing wheel mechanism 933. Further, at the sizing wheel mechanism 933 the forehead support is equipped with a support pad 932 forming a third forward face.

The first and second rail sections 9121, 9221 have a longitudinal opening in which a first guide member 915 and a second guide member 925, respectively, are slidably mounted. In particular, when not being fixed, the first and second guide members 915, 925 are slidable along the respective openings of the first and second rail sections 9121, 9221. The first coil unit 91 is mounted to the first guide member 915 by a first fastening screw 917 extending through a bore of the first guide member 915 and being screwed into a first screw socket 914 provided at a back side of the first coil unit 91. The second coil unit 92 is mounted to the second guide member 925 by a second fastening screw 927 extending through a bore of the second guide member 925 and being screwed into a second screw socket 924 provided at a back side of the second coil unit 91.

The first and second coil units 91, 92 have first and second housings 916, 926 inside each of which a litz wire is wound to form a coil. Further, the first coil unit 91 has a first forward face 911 and the second coil unit 92 has a second forward face 921, wherein the first and second forward faces 911, 921 are facing each other. To supply the coils of the first and second coil units 91, 92 with current, the first coil unit 91 is provided with a first current supply cable 918 and the second coil unit 92 is provided with a second current supply cable 928.

At a lower end side of the first rail section 9121 of the first frame portion 912, a first clavicula support bar 918 is pivotably mounted as support structure and at a lower end side of the second rail section 9221 of the second frame portion 922, a second clavicula support bar 928 is pivotably mounted as support structure. By being pivotably, the first and second clavicula support sections 918, 928 can be adapted to suit to a torso of a specific patient.

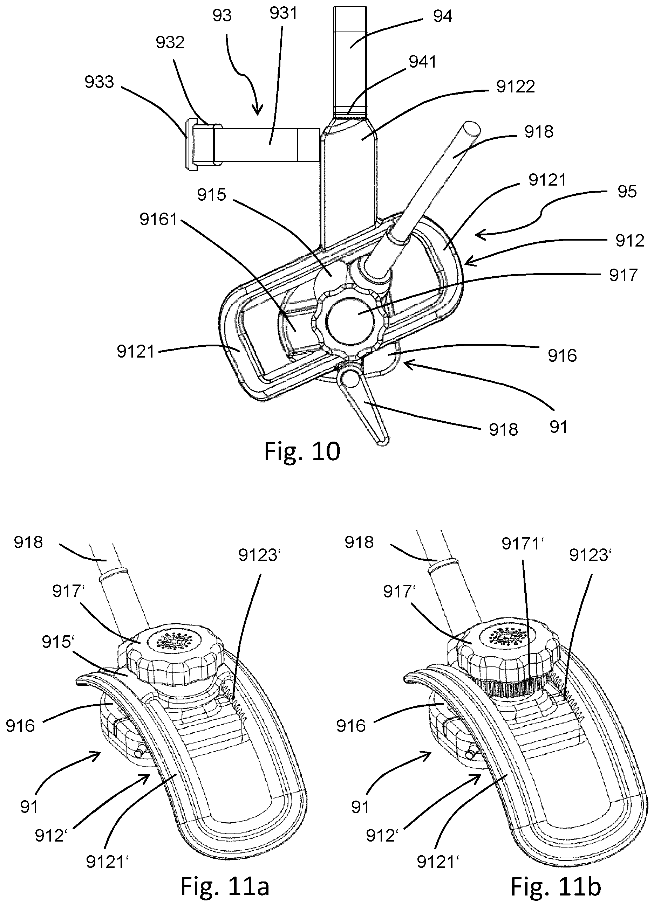

In Fig. 9 the fifth respiration promoting apparatus is shown in perspective, in which it can be seen that the overhead arch 94 is essentially perpendicular to the forehead support 93. Fig. 10 shows the fifth respiration promoting apparatus from a side.

In use of the fifth respiration promoting apparatus, the first and second height adjusting neck sections 9122, 9222 are adjusted in length such that the overhead arch lies on the head of the patient. In particular, the length is adjusted such that the first and second rails sections 9121, 92221 extend about the left and right sides of the neck of the patient. Since the rail sections are bent 9121, 9222 they can run along the neck. The forehead support 93 is also adjusted in length by turning the sizing wheel mechanism 933 such that the central support pad 932 abuts the forehead of the patient. The first and second support rods 918, 928 are adjusted such that they rest on the left and right clavicles of the torso of the patient. In this configuration, the respiration promoting apparatus is safely and stably mounted to the patient, wherein the two support rods 918, 928 and the forehead support 93 establish a robust three-point support.

Furthermore, the first and second guide members 915, 925 are slid along the first or second rail sections 9121, 9221 until the first and second forward faces 911, 921 of the first and second coil units 91, 92 are properly positioned at the neck of the patient to stimulate the two Phrenic nerves of the patient. Once being in an appropriate location and orientation, the first and second coil units 91, 92 are fixed to the first and second rail sections 9121, 9221 by tightening first and second fastening screws 917, 927. For allowing an efficient manual operation the first and second fastening screws 917, 927 are equipped with gripping portions. By tightening the first and second fastening screws 917, 927 the first and second coil units 91, 92 are outwardly pulled such that the respective rail section 9121 9221 is clamped between coil unit 91, 92 and guide member 915, 925. Like this, an efficient locking mechanism can be provided which allows to fix the respiration promoting apparatus in a status individually adjusted to the situation of the specific patient.

In Figs. 11a and 11b a portion of a sixth embodiment of a respiration promoting apparatus according to the invention is shown. In particular, Figs. 11a and 11b exemplarily depict the mounting of a first coil unit 91 to a first rail section 9121' of a first frame portion 912'. The sixth respiration promoting apparatus comprises a mirror symmetric coil second unit and mounting configuration. Furthermore, all elements of the sixth respiration promoting apparatus not shown and described to be different are identical to the fifth respiration promoting apparatus.

The first coil unit 91 is mounted to the first rail section 9121' via a first guide member 915'. At a border of an opening of the first rail section 9121' a toothing 9123' is provided. Further, as can be seen in Fig. 11b where the first guide member 915' is not depicted, a first screw 917' comprises a cogwheel 9171' positioned under the first guide member 915'. The cogwheel 9171' engages the toothing 9123'. By turning the first screw 917' the gearwheel 9171' travels along the toothing 9123' such that the first guide member 915' and the first coil unit 91 are moved along the first rail section 9121. Like this, the first coil unit 91 can conveniently be positioned at an appropriate location of the neck of the patient.

The scope of the invention is defined by the claims, whereas this description and the accompanying drawings illustrate aspects and embodiments of the present invention. In other words, while the invention has been illustrated and described in detail in the drawings and foregoing description, such illustration and description are to be considered illustrative or exemplary. Various mechanical, compositional, structural, electrical, and operational changes may be made without departing from the scope of this description and the claims. In some instances, well-known structures and techniques have not been shown in detail in order not to obscure the invention. Thus, it will be understood that changes and modifications may be made by those of ordinary skill within the scope of the following claims.

Furthermore, in the claims the word "comprising" does not exclude other elements or steps, and the indefinite article "a" or "an" does not exclude a plurality.

## Claims

1. An electro-magnetic respiration promoting apparatus to coordinately stimulate two Phrenic nerves (62) of a patient for activating a diaphragm of the patient, comprising:
a first coil unit (10; 91) having a first forward face (11; 911) configured to be positioned at the patient to stimulate a first Phrenic nerve of the two Phrenic nerves (62) of the patient;
a second coil unit (20; 92) having a second forward face (21; 921) configured to be positioned at the patient to stimulate a second Phrenic nerve of the two Phrenic nerves (62) of the patient; and
a bracket structure (30; 95) coupled to the first coil unit (10; 91) and the second coil unit (20; 92), wherein
the bracket structure (30; 95) is adjustable such that positions and orientations of the first coil unit (10; 91) and of the second coil unit (20; 92) are adapted,
the bracket structure (30; 95) has a third forward face (31; 918, 928, 932) configured to be positioned at the patient, and
the respiration promoting apparatus is configured to be arranged at a defined location of the patient when the first forward face (11; 911), the second forward face (21; 921) and the third forward face (31; 918, 928, 932) are positioned at the patient.

2. The respiration promoting apparatus of claim 1, wherein
the bracket structure (30; 95) comprises a first leg portion (12), a second leg portion (22) and a hinge portion (32) connecting the first leg portion (12) and the second leg portion (22) to each other,
the first coil unit (10; 91) is coupled to the first leg portion (12) and the second coil unit (20; 92) is coupled to the second leg portion (22), and
a distance (D) between the first coil unit (10; 91) and the second coil unit (20; 92) can be adjusted by pivoting the first leg portion (12) and the second leg portion (22) relative to each other about the hinge portion (32).

3. The respiration promoting apparatus of claim 2,
wherein the first leg portion (12) and/or the second leg portion (22) of the bracket structure (30; 95) are curved such that in any pivoting position of the first leg portion (12) and the second leg portion (22) relative to each other a free space (S) is provided between the hinge portion (32) and the first coil unit (10; 91) and/or the second coil unit (20; 92), and/or
wherein the first leg portion (12) and the second leg portion (22) of the bracket structure (30; 95) are curved such that a free space (S) is provided between the patient and the first leg portion (12) and/or between the patient and the second leg portion (22) when the respiration apparatus is arranged at the defined location of the patient, and/or
wherein the hinge portion (32) of the bracket structure (30; 95) comprises the third forward face (31; 918, 928, 932).

4. The respiration promoting apparatus of claim 2 or 3, wherein the bracket structure (30; 95) comprises a first pivoting coupler (33) extending from the first leg portion (12) and a second pivoting coupler (34) extending from the second leg portion (22), wherein the first coil unit (10; 91) is mounted to the first pivoting coupler (33) and the second coil unit (20; 92) is mounted to the second pivoting coupler (34).

5. The respiration promoting apparatus of claim 4,
wherein the first pivoting coupler (33) is pivotably mounted to the first leg portion (12) such that the first pivoting coupler (33) can be pivoted relative to the first leg portion (12) about an axis (A1) of the first leg portion (12), and respectively the second pivoting coupler (34) is pivotably mounted to the second leg portion (22) such that the second pivoting coupler (34) can be pivoted relative to the second leg portion (22) about an axis of the second leg portion (A2), and/or
wherein the first coil unit (10; 91) is pivotably mounted to the first pivoting coupler (33) such that the first coil unit (10; 91) can be pivoted relative to the first pivoting coupler (33) about an axis of the first pivoting coupler (A3), and the second coil unit (20; 92) is pivotably mounted to the second pivoting coupler (34) such that the second coil unit (20; 92) can be pivoted relative to the second pivoting coupler (34) about an axis (A4) of the second pivoting coupler (34),
wherein, preferably, the first axis (A1) along the first leg portion is essentially perpendicular to the first axis (A3) of the first pivoting coupler (33) and the second axis (A2) along the second leg portion (34) is essentially perpendicular to the second axis (A4) of the second pivoting coupler (34).

6. The respiration promoting apparatus of claim 4 or 5,
wherein
the first pivoting coupler (33) is Y-like or U-like shaped having a stem portion (35) and two branch portions (36), and the second pivoting coupler (34) is Y-like or U-like shaped having a stem portion (35') and two branch portions (36');
the stem portion (35) of first pivoting coupler (33) is mounted to the first leg portion (12), and the stem portion (35') of second pivoting coupler (34) is mounted to the second leg portion (22); and
the first coil unit (10; 91) is mounted between the two branch portions (36) of the first pivoting coupler (34), and the second coil unit (20; 92) is mounted between the two branch portions (36') of the second pivoting coupler (34), and/or wherein
the first pivoting coupler (33) is ring-shaped having a ring portion with a ring opening and a stem portion, and the second pivoting coupler (34) is ring-shaped having a ring portion with a ring opening and a stem portion;
the stem portion of first pivoting coupler (33) is mounted to the first leg portion (12), and the stem portion of second pivoting coupler (34) is mounted to the second leg portion (22); and
the first coil unit (10; 91) is mounted in the ring opening of the first pivoting coupler (33) and the second coil unit (20; 92) is mounted in the ring opening of the second pivoting coupler (34).

7. The respiration promoting apparatus of claim 1, wherein
the bracket structure (30; 95) comprises a first frame portion (912), a second frame portion (922) and a forehead support structure (93),
the first coil unit (10; 91) is coupled to the first frame portion (912) and the second coil unit (20; 92) is coupled to the second frame portion (922) (22),
the first frame portion (912) and the second frame portion (922) are connected via the forehead support structure (93), and
the forehead support structure (93) is configured to be positioned at and to contact a forehead of the patient.

8. The respiration promoting apparatus of claim 7, wherein the bracket structure (30; 95) comprises an overhead arch (94) having a first tangential end (941) and a second tangential end (942), the first frame portion (912) is mounted to the first tangential end (941) of the overhead arch (94), and the second frame portion (922) is mounted to the second tangential end (942) of the overhead arch (94), wherein, preferably, the bracket structure (30; 95) is configured such that a distance between the overhead arch (94) and the first coil unit (10; 91) and a distance between the overhead arch (94) and the second coil unit (20; 92) are adjustable.

9. The respiration promoting apparatus of claim 7 or 8,
wherein the forehead support structure (93) of the bracket structure (30; 95) is configured to be adjusted in accordance with a size of the forehead of the patient; and/or
wherein the forehead support structure (93) of the bracket structure (30; 95) comprises a band configured to extend along the forehead of the patient; and/or
wherein the forehead support structure (93) of the bracket structure (30; 95) comprises a pad configured to contact the forehead of the patient.

10. The respiration promoting apparatus of any one of claims 7 to 9, wherein each of the first frame portion (912) and the second frame portion (922) comprise a neck position guide section (9121, 9221) to which the first coil unit (10; 91) and the second coil unit (20; 92), respectively, is movably mounted such that the first coil unit (10; 91) and the second coil unit (20; 92) can be varyingly positioned about a neck of the patient,
wherein, preferably,
the first coil unit (10; 91) is mounted to the neck position guide section (9121, 9221) of the first frame portion (912) by a first fastening member (917) configured to be in a fixed state, in which the first coil unit (10; 91) is immovably fastened to the neck position guide section (9121, 9221) of the first frame portion (912), and in a loose state, in which the first coil unit (10; 91) is movable relative to the neck position guide section (9121, 9221) of the first frame portion (912), and
the second coil unit (20; 92) is mounted to the neck position guide section (9121, 9221) of the second frame portion (922) by a second fastening member (927) configured to be in a fixed state, in which the second coil unit (20; 92) is immovably fastened to the neck position guide section (9121, 9221) of the second frame portion (922), and in a loose state, in which the second coil unit (20; 92) is movable relative to the neck position guide section (9121, 9221) of the second frame portion (922).

11. The respiration promoting apparatus of any one of claims 7 to 10, wherein the bracket structure (30; 95) comprises two support structures (918, 928) configured to support the respiration promoting apparatus on a torso of the patient,
wherein, preferably, one of the two support structures is comprised by the first frame portion (912) and the other of the two support structures is comprised by the second frame portion (922), and/or
wherein, preferably, one of the two support structures comprises a first support bar (918) movably connected to the first frame portion (912) and the other of the two support structures comprises a second support bar (928) movably connected to the second frame portion (922).

12. The respiration promoting apparatus of any one of the preceding claims,
wherein a coil unit (10; 20) and/or the bracket structure (30; 95) comprises a vacuum device for fixing a forward face (11; 21; 31) to the patient using a vacuum; and/or
wherein the first coil unit (10; 91) has a non-flat first coil winding (13) formed from a conductive elongate component, and the second coil unit (20; 92) has a non-flat second coil winding (23) formed from a conductive elongate component; and/or
wherein the first coil unit (10; 91) and the second coil unit (20; 92) are detachably coupled to the bracket structure (30; 95); and/or
wherein the bracket structure (30; 95) or a control unit comprises a switch to direct current into either the first coil unit (10; 91) or the second coil unit (20; 92) to generate an alternating current supply in the coil units.

13. The respiration promoting apparatus of any one of the preceding claims, comprising a locking mechanism (38) to block the position and orientation of the first coil unit (10; 91) and the second coil unit (20; 92) relative to each other in a target configuration, wherein the locking mechanism (38) of the bracket structure (30; 95) preferably is configured to irreversibly block the position and orientation of the first coil unit (10; 91) and the second coil unit (20; 92) relative to each other in the target configuration.

14. The respiration promoting apparatus of any one of the preceding claims, wherein the backward face (17) of the first coil unit (10; 91) and the backward face (27) of the second coil unit (20; 92) are shielded,
wherein lateral faces of the first coil unit (10; 91) and the second coil unit (20; 92) preferably are shielded, and/or
preferably comprising an active shielding member configured to shield the backward faces (17, 27) of the first coil unit (10; 91) and the second coil unit (20; 92).

15. The respiration promoting apparatus of any one of the preceding claims, comprising a first biofeedback sensor (18), a second biofeedback sensor (28) and a control unit, wherein
the first biofeedback sensor (18) is associated to the first coil unit (10; 91) and coupled to the control unit,
the second biofeedback sensor (28) is associated to the second coil unit (20; 92) and coupled to the control unit, and
the control unit is configured to individually provide current to the first coil unit (10; 91) and to the second coil unit (20; 92) in accordance with signals received from the first biofeedback sensor (18) and the second biofeedback sensor (28) such that the diaphragm is uniformly activated.

## Patentansprüche

1. Atmungsförderungsgerät zur koordinierten Stimulation zweier Phrenicusnerven (62) eines Patienten zur Aktivierung des Zwerchfells des Patienten, umfassend:
eine erste Spuleneinheit (10; 91) mit einer ersten Vorderseite (11; 911), die so ausgebildet ist, dass sie am Patienten positioniert werden kann, um einen ersten Phrenicusnerv der beiden Phrenicusnerven (62) des Patienten zu stimulieren;
eine zweite Spuleneinheit (20; 92) mit einer zweiten Vorderseite (21; 921), die so ausgebildet ist, dass sie am Patienten positioniert werden kann, um einen zweiten Nervus phrenicus der beiden Phrenicusnerven (62) des Patienten zu stimulieren; und
eine Halterungsstruktur (30; 95), die mit der ersten Spuleneinheit (10; 91) und der zweiten Spuleneinheit (20; 92) verbunden ist, wobei
die Halterungsstruktur (30; 95) so einstellbar ist, dass Positionen und Ausrichtungen der ersten Spuleneinheit (10; 91) und der zweiten Spuleneinheit (20; 92) angepasst werden,
die Halterungsstruktur (30; 95) eine dritte Vorderseite (31; 918, 928, 932) aufweist, die so ausgebildet ist, dass sie am Patienten positioniert werden kann, und
das Atmungsförderungsgerät so ausgebildet ist, dass es an einer definierten Stelle des Patienten angeordnet wird, wenn die erste Vorderseite (11; 911), die zweite Vorderseite (21; 921) und die dritte Vorderseite (31; 918, 928, 932) am Patienten positioniert sind.

2. Atmungsförderungsgerät nach Anspruch 1, wobei
die Halterungskonstruktion (30; 95) einen ersten Schenkelabschnitt (12), einen zweiten Schenkelabschnitt (22) und einen Gelenkabschnitt (32) umfasst, der den ersten Schenkelabschnitt (12) und den zweiten Schenkelabschnitt (22) miteinander verbindet,
die erste Spuleneinheit (10; 91) mit dem ersten Schenkelabschnitt (12) und die zweite Spuleneinheit (20; 92) mit dem zweiten Schenkelabschnitt (22) verbunden ist, und
ein Abstand (D) zwischen der ersten Spuleneinheit (10; 91) und der zweiten Spuleneinheit (20; 92) durch Schwenken des ersten Schenkelabschnitts (12) und des zweiten Schenkelabschnitts (22) relativ zueinander um den Gelenkabschnitt (32) eingestellt werden kann.

3. Atmungsförderungsgerät nach Anspruch 2,
wobei der erste Schenkelabschnitt (12) und/oder der zweite Schenkelabschnitt (22) der Halterungsstruktur (30; 95) derart gekrümmt sind, dass in jeder Schwenkposition des ersten Schenkelabschnitts (12) und des zweiten Schenkelabschnitts (22) relativ zueinander ein Freiraum (S) zwischen dem Gelenkabschnitt (32) und der ersten Spuleneinheit (10; 91) und/oder der zweiten Spuleneinheit (20; 92) vorhanden ist, und/oder
wobei der erste Schenkelabschnitt (12) und der zweite Schenkelabschnitt (22) der Halterungsstruktur (30; 95) derart gekrümmt sind, dass ein Freiraum (S) zwischen dem Patienten und dem ersten Schenkelabschnitt (12) und/oder zwischen dem Patienten und dem zweiten Schenkelabschnitt (22) bereitgestellt wird, wenn das Beatmungsgerät an der definierten Stelle des Patienten angeordnet ist, und/oder
wobei der Gelenkabschnitt (32) der Halterungsstruktur (30; 95) die dritte Vorderseite (31; 918, 928, 932) umfasst.

4. Atmungsförderungsgerät nach Anspruch 2 oder 3, wobei die Halterungsstruktur (30; 95) eine erste Schwenkkupplung (33), die sich vom ersten Schenkelabschnitt (12) erstreckt, und eine zweite Schwenkkupplung (34), die sich vom zweiten Schenkelabschnitt (22) erstreckt, umfasst, wobei die erste Spuleneinheit (10; 91) an der ersten Schwenkkupplung (33) und die zweite Spiraleinheit (20; 92) an der zweiten Schwenkkupplung Schwenkgelenk (34) angebracht ist.

5. Atmungsförderungsgerät nach Anspruch 4,
wobei die erste Schwenkkupplung (33) schwenkbar an dem ersten Schenkelabschnitt (12) angebracht ist, so dass die erste Schwenkkupplung (33) relativ zu dem ersten Schenkelabschnitt (12) um eine Achse (A1) des ersten Schenkelabschnitts (12) geschwenkt werden kann, und entsprechend die zweite Schwenkkupplung (34) schwenkbar an dem zweiten Schenkelabschnitt (22) angebracht ist, sodass die zweite Schwenkkupplung (34) relativ zu dem zweiten Schenkelabschnitt (22) um eine Achse des zweiten Schenkelabschnitts (A2) geschwenkt werden kann, und/oder
wobei die erste Spuleneinheit (10; 91) schwenkbar an dem ersten Schwenkgelenk (33) angebracht ist, sodass die erste Spuleneinheit (10; 91) relativ zu dem ersten Schwenkgelenk (33) um eine Achse des ersten Schwenkgelenks (A3) geschwenkt werden kann, und die zweite Spuleneinheit (20; 92) schwenkbar an dem zweiten Schwenkgelenk (34) angebracht ist, so dass die zweite Spuleneinheit (20; 92) relativ zu dem zweiten Schwenkgelenk (34) um eine Achse (A4) des zweiten Schwenkgelenks (34) geschwenkt werden kann,
wobei vorzugsweise die erste Achse (A1) entlang des ersten Schenkelabschnitts im Wesentlichen senkrecht zur ersten Achse (A3) des ersten Schwenkgelenks (33) steht und die zweite Achse (A2) entlang des zweiten Schenkelabschnitts (34) im Wesentlichen senkrecht zur zweiten Achse (A4) des zweiten Schwenkgelenks (34) steht.

6. Atmungsförderungsgerät nach Anspruch 4 oder 5,
wobei
die erste Schwenkkupplung (33) Y- oder U-förmig ausgebildet ist und einen Schaftabschnitt (35) sowie zwei Abzweigabschnitte (36) aufweist, und die zweite Schwenkkupplung (34) Y- oder U-förmig ausgebildet ist und einen Schaftabschnitt (35') sowie zwei Abzweigabschnitte (36') aufweist;
der Schaftabschnitt (35) der ersten Schwenkkupplung (33) am ersten Schenkelabschnitt (12) angebracht ist und der Schaftabschnitt (35') der zweiten Schwenkkupplung (34) am zweiten Schenkelabschnitt (22) angebracht ist; und
die erste Spuleneinheit (10; 91) zwischen den beiden Abzweigabschnitten (36) der ersten Schwenkkupplung (34) angebracht ist und die zweite Spuleneinheit (20; 92) zwischen den beiden Abzweigabschnitten (36') der zweiten Schwenkkupplung (34) angebracht ist, und/oder wobei
die erste Schwenkkupplung (33) ringförmig ist und einen Ringabschnitt mit einer Ringöffnung sowie einen Schaftabschnitt aufweist, und die zweite Schwenkkupplung (34) ringförmig ist und einen Ringabschnitt mit einer Ringöffnung sowie einen Schaftabschnitt aufweist;
der Schaftabschnitt der ersten Schwenkkupplung (33) am ersten Schenkelabschnitt (12) angebracht ist und der Schaftabschnitt der zweiten Schwenkkupplung (34) am zweiten Schenkelabschnitt (22) angebracht ist; und
die erste Spuleneinheit (10; 91) in der Ringöffnung der ersten Schwenkkupplung (33) angebracht ist und die zweite Spuleneinheit (20; 92) in der Ringöffnung der zweiten Schwenkkupplung (34) angebracht ist.

7. Atmungsförderungsgerät nach Anspruch 1, wobei
die Halterungsstruktur (30; 95) einen ersten Rahmenabschnitt (912), einen zweiten Rahmenabschnitt (922) und eine Stirnstützstruktur (93) umfasst,
die erste Spuleneinheit (10; 91) mit dem ersten Rahmenabschnitt (912) verbunden ist und die zweite Spuleneinheit (20; 92) mit dem zweiten Rahmenabschnitt (922) (22) verbunden ist,
der erste Rahmenabschnitt (912) und der zweite Rahmenabschnitt (922) über die Stirnstützstruktur (93) verbunden sind und
die Stirnstützstruktur (93) so ausgebildet ist, dass sie an der Stirn des Patienten positioniert wird und diese berührt.

8. Atmungsförderungsgerät nach Anspruch 7, wobei die Halterungsstruktur (30; 95) einen Überkopfbogen (94) mit einem ersten tangentialen Ende (941) und einem zweiten tangentialen Ende (942) umfasst, der erste Rahmenabschnitt (912) am ersten tangentialen Ende (941) des Überkopfbogens (94) angebracht ist, und der zweite Rahmenabschnitt (922) am zweiten tangentialen Ende (942) des Überkopfbogens (94) angebracht ist, wobei die Halterungsstruktur (30; 95) vorzugsweise so ausgebildet ist, dass ein Abstand zwischen dem Überkopfbogen (94) und der ersten Spuleneinheit (10; 91) sowie ein Abstand zwischen dem Überkopfbogen (94) und der zweiten Spuleneinheit (20; 92) einstellbar sind.

9. Atmungsförderungsgerät nach Anspruch 7 oder 8,
wobei die Stirnstützstruktur (93) der Halterungsstruktur (30; 95) so ausgebildet ist, dass sie entsprechend der Größe der Stirn des Patienten verstellbar ist; und/oder
wobei die Stirnstützstruktur (93) der Halterungsstruktur (30; 95) ein Band umfasst, das so ausgebildet ist, dass es sich entlang der Stirn des Patienten erstreckt; und/oder
wobei die Stirnstützstruktur (93) der Halterungsstruktur (30; 95) ein Polster umfasst, das so ausgebildet ist, um die Stirn des Patienten zu kontaktieren.

10. Atmungsförderungsgerät nach einem der Ansprüche 7 bis 9, wobei sowohl der erste Rahmenabschnitt (912) als auch der zweite Rahmenabschnitt (922) jeweils einen Halspositionsführungsabschnitt (9121, 9221) umfassen, an dem die erste Spuleneinheit (10; 91) bzw. die zweite Spuleneinheit (20; 92) beweglich angebracht ist, so dass die erste Spuleneinheit (10; 91) und die zweite Spuleneinheit (20; 92) variabel um den Hals des Patienten positioniert werden können,
wobei vorzugsweise
die erste Spuleneinheit (10; 91) an dem Halspositionsführungsabschnitt (9121, 9221) des ersten Rahmenabschnitts (912) mittels eines ersten Befestigungselements (917) angebracht ist, das so ausgebildet ist, um sich in einem festen Zustand zu befinden, in dem die erste Spuleneinheit (10; 91) unbeweglich an dem Halspositionsführungsabschnitt (9121, 9221) des ersten Rahmenabschnitts (912) befestigt ist, und in einem losen Zustand, in dem die erste Spuleneinheit (10; 91) relativ zu dem Halspositionsführungsabschnitt (9121, 9221) des ersten Rahmenabschnitts (912) beweglich ist, und
die zweite Spuleneinheit (20; 92) an dem Halspositionsführungsabschnitt (9121, 9221) des zweiten Rahmenabschnitts (922) mittels eines zweiten Befestigungselements (927) angebracht ist, das so ausgebildet ist, um sich in einem festen Zustand zu befinden, in dem die zweite Spuleneinheit (20; 92) unbeweglich am Halspositionsführungsabschnitt (9121, 9221) des zweiten Rahmenteils (922) befestigt ist, und in einem losen Zustand, in dem die zweite Spuleneinheit (20; 92) relativ zum Halspositionsführungsabschnitt (9121, 9221) des zweiten Rahmenteils (922) beweglich ist.

11. Atmungsförderungsgerät nach einem der Ansprüche 7 bis 10,
wobei die Halterungsstruktur (30; 95) zwei Stützstrukturen (918, 928) umfasst, die so ausgebildet sind, um das Atmungsförderungsgerät am Oberkörper des Patienten abzustützen,
wobei vorzugsweise eine der beiden Stützstrukturen von dem ersten Rahmenabschnitt (912) und die andere der beiden Stützstrukturen von dem zweiten Rahmenabschnitt (922) umfasst ist, und/oder
wobei vorzugsweise eine der beiden Stützstrukturen eine erste Stützstange (918) umfasst, die beweglich mit dem ersten Rahmenabschnitt (912) verbunden ist, und die andere der beiden Stützstrukturen eine zweite Stützstange (928) umfasst, die beweglich mit dem zweiten Rahmenabschnitt (922) verbunden ist.

12. Atmungsförderungsgerät nach einem der vorstehenden Ansprüche,
wobei eine Spuleneinheit (10; 20) und/oder die Halterungskonstruktion (30; 95) eine Vakuumvorrichtung umfasst, um eine Vorderseite (11; 21; 31) durch Vakuum am Patienten zu befestigen; und/oder
wobei die erste Spuleneinheit (10; 91) eine nicht flache erste Spulenwicklung (13) aufweist, die aus einem leitfähigen, länglichen Bauteil gebildet ist, und die zweite Spuleneinheit (20; 92) eine nicht flache zweite Spulenwicklung (23) aufweist, die aus einem leitfähigen, länglichen Bauteil gebildet ist; und/oder
wobei die erste Spuleneinheit (10; 91) und die zweite Spuleneinheit (20; 92) lösbar mit der Halterungsstruktur (30; 95) verbunden sind; und/oder
wobei die Halterungsstruktur (30; 95) oder eine Steuereinheit einen Schalter umfasst, um Strom entweder in die erste Spuleneinheit (10; 91) oder in die zweite Spuleneinheit (20; 92) zu leiten, um in den Spuleneinheiten eine Wechselstromversorgung zu erzeugen.

13. Atmungsförderungsgerät nach einem der vorstehenden Ansprüche, umfassend einen Verriegelungsmechanismus (38), um die Position und Ausrichtung der ersten Spuleneinheit (10; 91) und der zweiten Spuleneinheit (20; 92) relativ zueinander in einer Zielkonfiguration zu arretieren, wobei der Verriegelungsmechanismus (38) der Halterungsstruktur (30; 95) vorzugsweise so ausgebildet ist, dass er die Position und Ausrichtung der ersten Spuleneinheit (10; 91) und der zweiten Spuleneinheit (20; 92) relativ zueinander in der Zielkonfiguration irreversibel arretiert.

14. Atmungsförderungsgerät nach einem der vorstehenden Ansprüche, wobei die Rückseite (17) der ersten Spuleneinheit (10; 91) und die Rückseite (27) der zweiten Spuleneinheit (20; 92) abgeschirmt sind,
wobei vorzugsweise die Seitenflächen der ersten Spuleneinheit (10; 91) und der zweiten Spuleneinheit (20; 92) abgeschirmt sind, und/oder
vorzugsweise umfassend ein aktives Abschirmelement, das so ausgebildet ist, dass es die Rückseiten (17, 27) der ersten Spuleneinheit (10; 91) und der zweiten Spuleneinheit (20; 92) abschirmt.

15. Atmungsförderungsgerät nach einem der vorstehenden Ansprüche, umfassend einen ersten Biofeedback-Sensor (18), einen zweiten Biofeedback-Sensor (28) und eine Steuereinheit, wobei
der erste Biofeedback-Sensor (18) der ersten Spuleneinheit (10; 91) zugeordnet und mit der Steuereinheit gekoppelt ist,
der zweite Biofeedback-Sensor (28) der zweiten Spuleneinheit (20; 92) zugeordnet und mit der Steuereinheit gekoppelt ist, und
die Steuereinheit so konfiguriert ist, dass sie die erste Spuleneinheit (10; 91) und die zweite Spuleneinheit (20; 92) entsprechend den vom ersten Biofeedback-Sensor (18) und vom zweiten Biofeedback-Sensor (28) empfangenen Signalen individuell mit Strom versorgt, sodass das Zwerchfell gleichmäßig aktiviert wird.

## Revendications

1. Appareil d'aide respiratoire électromagnétique permettant de stimuler de manière coordonnée deux nerfs phréniques (62) d'un patient pour activer un diaphragme du patient, comprenant :
une première unité de bobine (10 ; 91) ayant une première face avant (11 ; 911) conçue pour être positionnée au niveau du patient pour stimuler un premier nerf phrénique des deux nerfs phréniques (62) du patient ;
une seconde unité de bobine (20 ; 92) ayant une deuxième face avant (21 ; 921) conçue pour être positionnée au niveau du patient pour stimuler un second nerf phrénique des deux nerfs phréniques (62) du patient ; et
une structure de support (30 ; 95) accouplée à la première unité de bobine (10 ; 91) et à la seconde unité de bobine (20 ; 92), dans lequel
la structure de support (30 ; 95) est ajustable de manière que des positions et des orientations de la première unité de bobine (10 ; 91) et de la seconde unité de bobine (20 ; 92) soient adaptées,
la structure de support (30 ; 95) a une troisième face avant (31 ; 918, 928, 932) conçue pour être positionnée au niveau du patient, et
l'appareil d'aide respiratoire est conçu pour être placé au niveau d'un emplacement défini du patient lorsque la première face avant (11 ; 911), la deuxième face avant (21 ; 921) et la troisième face avant (31 ; 918, 928, 932) sont positionnées au niveau du patient.

2. Appareil d'aide respiratoire selon la revendication 1, dans lequel
la structure de support (30 ; 95) comprend une première partie bras (12), une seconde partie bras (22) et une partie charnière (32) reliant la première partie bras (12) et la seconde partie bras (22) l'une à l'autre,
la première unité de bobine (10 ; 91) est accouplée à la première partie bras (12) et la seconde unité de bobine (20 ; 92) est accouplée à la seconde partie bras (22), et
une distance (D) entre la première unité de bobine (10 ; 91) et la seconde unité de bobine (20 ; 92) peut être ajustée en faisant pivoter la première partie bras (12) et la seconde partie bras (22) l'une par rapport à l'autre autour de la partie charnière (32).

3. Appareil d'aide respiratoire selon la revendication 2,
dans lequel la première partie bras (12) et/ou la seconde partie bras (22) de la structure de support (30 ; 95) sont incurvées de manière que dans n'importe quelle position de pivotement de la première partie bras (12) et de la seconde partie bras (22) l'une par rapport à l'autre, un espace libre (S) soit ménagé entre la partie charnière (32) et la première unité de bobine (10 ; 91) et/ou la seconde unité de bobine (20 ; 92), et/ou
dans lequel la première partie bras (12) et la seconde partie bras (22) de la structure de support (30 ; 95) sont incurvées de manière qu'un espace libre (S) soit ménagé entre le patient et la première partie bras (12) et/ou entre le patient et la seconde partie bras (22) lorsque l'appareil respiratoire est placé à l'emplacement défini du patient, et/ou
dans lequel la partie charnière (32) de la structure de support (30 ; 95) comprend la troisième face avant (31 ; 918, 928, 932).

4. Appareil d'aide respiratoire selon la revendication 2 ou 3, dans lequel la structure de support (30 ; 95) comprend un premier coupleur pivotant (33) s'étendant à partir de la première partie bras (12) et un second coupleur pivotant (34) s'étendant à partir de la seconde partie bras (22), dans lequel la première unité de bobine (10 ; 91) est montée sur le premier coupleur pivotant (33) et la seconde unité de bobine (20 ; 92) est montée sur le second coupleur pivotant (34).

5. Appareil d'aide respiratoire selon la revendication 4,
dans lequel le premier coupleur pivotant (33) est monté pivotant sur la première partie bras (12) de manière que le premier coupleur pivotant (33) puisse pivoter par rapport à la première partie bras (12) autour d'un axe (A1) de la première partie bras (12), et respectivement le second coupleur pivotant (34) est monté pivotant sur la seconde partie bras (22) de manière que le second coupleur pivotant (34) puisse pivoter par rapport à la seconde partie bras (22) autour d'un axe de la seconde partie bras (A2), et/ou
dans lequel la première unité de bobine (10 ; 91) est montée pivotante sur le premier coupleur pivotant (33) de manière que la première unité de bobine (10 ; 91) puisse pivoter par rapport au premier coupleur pivotant (33) autour d'un axe du premier coupleur pivotant (A3), et la seconde unité de bobine (20 ; 92) est montée pivotante sur le second coupleur pivotant (34) de manière que la seconde unité de bobine (20 ; 92) puisse pivoter par rapport au second coupleur pivotant (34) autour d'un axe (A4) du second coupleur pivotant (34),
dans lequel, de préférence, le premier axe (A1) le long de la première partie bras est essentiellement perpendiculaire au premier axe (A3) du premier coupleur pivotant (33) et le second axe (A2) le long de la seconde partie bras (34) est essentiellement perpendiculaire au second axe (A4) du second coupleur pivotant (34).

6. Appareil d'aide respiratoire selon la revendication 4 ou 5,
dans lequel
le premier coupleur pivotant (33) est en forme de Y ou de U ayant une partie tige (35) et deux parties branche (36), et le second coupleur pivotant (34) est en forme de Y ou de U ayant une partie tige (35') et deux parties branche (36') ;
la partie tige (35) du premier coupleur pivotant (33) est montée sur la première partie bras (12), et la partie tige (35') du second coupleur pivotant (34) est montée sur la seconde partie bras (22) ; et
la première unité de bobine (10 ; 91) est montée entre les deux parties branche (36) du premier coupleur pivotant (34), et la seconde unité de bobine (20 ; 92) est montée entre les deux parties branche (36') du second coupleur pivotant (34), et/ou dans lequel
le premier coupleur pivotant (33) est en forme d'anneau ayant une partie annulaire avec une ouverture annulaire et une partie tige, et le second coupleur pivotant (34) est en forme d'anneau ayant une partie annulaire avec une ouverture annulaire et une partie tige ;
la partie tige du premier coupleur pivotant (33) est montée sur la première partie bras (12), et la partie tige du second coupleur pivotant (34) est montée sur la seconde partie bras (22) ; et
la première unité de bobine (10 ; 91) est montée dans l'ouverture annulaire du premier coupleur pivotant (33) et la seconde unité de bobine (20 ; 92) est montée dans l'ouverture annulaire du second coupleur pivotant (34).

7. Appareil d'aide respiratoire selon la revendication 1, dans lequel
la structure de support (30 ; 95) comprend une première partie cadre (912), une seconde partie cadre (922) et une structure de support frontale (93),
la première unité de bobine (10 ; 91) est accouplée à la première partie cadre (912) et la seconde unité de bobine (20 ; 92) est accouplée à la seconde partie cadre (922) (22),
la première partie cadre (912) et la seconde partie cadre (922) sont reliées par l'intermédiaire de la structure de support frontale (93), et
la structure de support frontale (93) est conçue pour être positionnée au niveau du front du patient et pour entrer en contact avec celui-ci.

8. Appareil d'aide respiratoire selon la revendication 7, dans lequel la structure de support (30 ; 95) comprend un arc supérieur (94) ayant une première extrémité tangentielle (941) et une seconde extrémité tangentielle (942), la première partie cadre (912) est montée sur la première extrémité tangentielle (941) de l'arc supérieur (94), et la seconde partie cadre (922) est montée sur la seconde extrémité tangentielle (942) de l'arc supérieur (94), dans lequel, de préférence, la structure de support (30 ; 95) est conçue de manière qu'une distance entre l'arc supérieur (94) et la première unité de bobine (10 ; 91) et une distance entre l'arc supérieur (94) et la seconde unité de bobine (20 ; 92) soient ajustables.

9. Appareil d'aide respiratoire selon la revendication 7 ou 8,
dans lequel la structure de support frontale (93) de la structure de support (30 ; 95) est conçue pour être ajustée en fonction d'une taille du front du patient ; et/ou
dans lequel la structure de support frontale (93) de la structure de support (30 ; 95) comprend une bande conçue pour s'étendre le long du front du patient ; et/ou
dans lequel la structure de support frontale (93) de la structure de support (30 ; 95) comprend un coussinet conçu pour entrer en contact avec le front du patient.

10. Appareil d'aide respiratoire selon l'une quelconque des revendications 7 à 9, dans lequel chacune de la première partie cadre (912) et de la seconde partie cadre (922) comprend une section de guidage de position de cou (9121, 9221) sur laquelle la première unité de bobine (10 ; 91) et la seconde unité de bobine (20 ; 92), respectivement, sont montées mobiles de manière que la première unité de bobine (10 ; 91) et la seconde unité de bobine (20 ; 92) puissent être positionnées de manière variable autour d'un cou du patient,
dans lequel, de préférence,
la première unité de bobine (10 ; 91) est montée sur la section de guidage de position de cou (9121, 9221) de la première partie cadre (912) par un premier élément de fixation (917) conçu pour être dans un état fixe, dans lequel la première unité de bobine (10 ; 91) est fixée à demeure à la section de guidage de position de cou (9121, 9221) de la première partie cadre (912), et dans un état lâche, dans lequel la première unité de bobine (10 ; 91) est mobile par rapport à la section de guidage de position de cou (9121, 9221) de la première partie cadre (912), et
la seconde unité de bobine (20 ; 92) est montée sur la section de guidage de position de cou (9121, 9221) de la seconde partie cadre (922) par un second élément de fixation (927) conçu pour être dans un état fixe, dans lequel la seconde unité de bobine (20 ; 92) est fixée à demeure à la section de guidage de position de cou (9121, 9221) de la seconde partie cadre (922), et dans un état lâche, dans lequel la seconde unité de bobine (20 ; 92) est mobile par rapport à la section de guidage de position de cou (9121, 9221) de la seconde partie cadre (922).

11. Appareil d'aide respiratoire selon l'une quelconque des revendications 7 à 10, dans lequel la structure de support (30 ; 95) comprend deux structures de support (918, 928) conçues pour supporter l'appareil d'aide respiratoire sur le torse du patient,
dans lequel, de préférence, l'une des deux structures de support est constituée par la première partie cadre (912) et l'autre des deux structures de support est constituée par la seconde partie cadre (922), et/ou
dans lequel, de préférence, l'une des deux structures de support comprend une première barre de support (918) reliée mobile à la première partie cadre (912) et l'autre des deux structures de support comprend une seconde barre de support (928) reliée mobile à la seconde partie cadre (922).

12. Appareil d'aide respiratoire selon l'une quelconque des revendications précédentes,
dans lequel une unité de bobine (10 ; 20) et/ou la structure de support (30 ; 95) comprennent un dispositif à vide pour fixer une face avant (11 ; 21 ; 31) au patient à l'aide d'un vide ; et/ou
dans lequel la première unité de bobine (10 ; 91) possède un premier enroulement de bobine non plat (13) formé à partir d'un composant allongé conducteur, et la seconde unité de bobine (20 ; 92) possède un second enroulement de bobine non plat (23) formé à partir d'un composant allongé conducteur ; et/ou
dans lequel la première unité de bobine (10 ; 91) et la seconde unité de bobine (20 ; 92) sont accouplées amovibles à la structure de support (30 ; 95) ; et/ou
dans lequel la structure de support (30 ; 95) ou une unité de commande comprend un commutateur pour diriger un courant vers la première unité de bobine (10 ; 91) ou la seconde unité de bobine (20 ; 92) pour générer une alimentation en courant alternatif dans les unités de bobine.

13. Appareil d'aide respiratoire selon l'une quelconque des revendications précédentes, comprenant un mécanisme de verrouillage (38) permettant de bloquer la position et l'orientation de la première unité de bobine (10 ; 91) et de la seconde unité de bobine (20 ; 92) l'une par rapport à l'autre dans une configuration cible, dans lequel le mécanisme de verrouillage (38) de la structure de support (30 ; 95) est de préférence conçu pour bloquer de manière irréversible la position et l'orientation de la première unité de bobine (10 ; 91) et de la seconde unité de bobine (20 ; 92) l'une par rapport à l'autre dans la configuration cible.

14. Appareil d'aide respiratoire selon l'une quelconque des revendications précédentes, dans lequel la face arrière (17) de la première unité de bobine (10 ; 91) et la face arrière (27) de la seconde unité de bobine (20 ; 92) sont protégées,
dans lequel des faces latérales de la première unité de bobine (10 ; 91) et de la seconde unité de bobine (20 ; 92) sont de préférence protégées, et/ou
comprenant de préférence un élément de protection actif conçu pour protéger les faces arrière (17, 27) de la première unité de bobine (10 ; 91) et de la seconde unité de bobine (20 ; 92).

15. Appareil d'aide respiratoire selon l'une quelconque des revendications précédentes, comprenant un premier capteur de rétroaction biologique (18), un second capteur de rétroaction biologique (28) et une unité de commande, dans lequel
le premier capteur de rétroaction biologique (18) est associé à la première unité de bobine (10 ; 91) et accouplé à l'unité de commande,
le second capteur de rétroaction biologique (28) est associé à la seconde unité de bobine (20 ; 92) et accouplé à l'unité de commande, et
l'unité de commande est conçue pour fournir individuellement un courant à la première unité de bobine (10 ; 91) et à la seconde unité de bobine (20 ; 92) conformément à des signaux reçus à partir du premier capteur de rétroaction biologique (18) et du second capteur de rétroaction biologique (28) de manière que le diaphragme soit activé uniformément.
